# EUROPÄISCHE PATENTSCHRIFT

(11) **EP 1 993 982 B1**
(45) Veröffentlichungstag und Bekanntmachung des Hinweises auf die Patenterteilung: **09.03.2016**
(21) Anmeldenummer: 06830695.0
(22) Anmeldetag: 18.12.2006
(51) Int. Cl.: C07C 5/32, C07C 5/333, B01J 19/02, C22C 38/40

(54) **VERFAHREN DER KONTINUIERLICHEN HETEROGEN KATALYSIERTEN PARTIELLEN DEHYDRIERUNG WENIGSTENS EINES ZU DEHYDRIERENDEN KOHLENWASSERSTOFFS**
METHOD FOR THE CONTINUOUS HETEROGENEOUSLY CATALYSED PARTIAL DEHYDROGENATION OF AT LEAST ONE HYDROCARBON TO BE DEHYDROGENATED
PROCEDE DE DESHYDROGENATION PARTIELLE, HETEROGENE, CONTINUE ET CATALYSEE D'AU MOINS UN HYDROCARBURE A DESHYDROGENER

(30) Priorität: 21.12.2005 DE 102005061626; 21.12.2005 US 751973 P
(43) Veröffentlichungstag der Anmeldung: 26.11.2008
(73) Patentinhaber: BASF SE, 67056 Ludwigshafen (DE)
(72) Erfinder: HECHLER, Claus, 67063 Ludwigshafen (DE); RUPPEL, Wilhelm, 68163 Mannheim (DE); SCHINDLER, Götz-Peter, 67071 Ludwigshafen (DE); KLANNER, Catharina, 68163 Mannheim (DE); BASSLER, Hans-Jürgen, 67433 Neustadt (DE); DIETERLE, Martin, 67063 Ludwigshafen (DE); KLAPPERT, Karl-Heinrich, 67134 Birkenheide (DE); MÜLLER-ENGEL, Klaus Joachim, 76297 Stutensee (DE)
(86) Internationale Anmeldenummer: PCT/EP2006/069859
(87) Internationale Veröffentlichungsnummer: WO 2007/071654

(56) Entgegenhaltungen:
- EP-A1- 0 718 415
- FR-A1- 2 175 755
- US-A1- 2003 044 334

## Beschreibung

Vorliegende Erfindung betrifft ein Verfahren der kontinuierlichen heterogen katalysierten partiellen Dehydrierung wenigstens eines zu dehydrierenden Kohlenwasserstoffs in der Gasphase, umfassend eine Verfahrensweise, bei der man einem Reaktionsraum, der von einer den Reaktionsraum berührenden (materiellen) Einhüllenden umschlossen ist, die wenigstens eine erste Öffnung zur Zufuhr wenigstens eines Ausgangsgasstroms in den Reaktionsraum und wenigstens eine zweite Öffnung zur Entnahme wenigstens eines Produktgasstroms aus dem Reaktionsraum aufweist,
- wenigstens einen, wenigstens einen zu dehydrierenden Kohlenwasserstoff enthaltenden, Ausgangsgasstrom kontinuierlich zuführt,
- im Reaktionsraum den wenigstens einen zu dehydrierenden Kohlenwasserstoff durch wenigstens ein im Reaktionsraum befindliches Katalysatorbett führt, und unter Erzeugung eines den wenigstens einen dehydrierten Kohlenwasserstoff, nicht umgesetzten zu dehydrierenden Kohlenwasserstoff sowie molekularen Wasserstoff und/oder Wasserdampf enthaltenden Produktgases oxidativ oder nicht oxidativ zu wenigstens einem dehydrierten Kohlenwasserstoff partiell dehydriert, und
- dem Reaktionsraum wenigstens einen Produktgasstrom kontinuierlich entnimmt.

Außerdem betrifft vorliegende Erfindung eine Vorrichtung zur Durchführung des erfindungsgemäßen Verfahrens sowie Verfahren der Partialoxidation des wenigstens einen dehydrierten Kohlenwasserstoffs.

Der in dieser Anmeldung verwendete Begriff "dehydrierter Kohlenwasserstoff" soll Kohlenwasserstoffe umfassen, deren Moleküle wenigstens zwei ("zwei" sind anwendungstechnisch bevorzugt) Wasserstoffatome weniger enthalten, als die Moleküle eines zu dehydrierenden Kohlenwasserstoffs. Im übrigen soll der Begriff Kohlenwasserstoff Stoffe umfassen, deren Moleküle nur aus den Elementen Kohlenstoff und Wasserstoff aufgebaut ist.

Damit umfassen dehydrierte Kohlenwasserstoffe insbesondere acyclische und cyclische aliphatische Kohlenwasserstoffe mit einer oder mehreren C, C-Doppelbindungen im Molekül.

Beispiele für solche aliphatischen dehydrierten Kohlenwasserstoffe sind Propen, iso-Buten, Ethylen, 1-Buten, 2-Buten und Butadien. D.h., zu den dehydrierten Kohlenwasserstoffen gehören insbesondere die einfach ungesättigten linearen (n-Alkene) oder verzweigten aliphatischen Kohlenwasserstoffe (z. B. iso-Alkene), sowie die Cycloalkene. Ferner sollen die dehydrierten Kohlenwasserstoffe auch die Alkapolyene (z. B. Diene und Triene) umfassen, die mehr als eine Kohlenstoff-Kohlenstoff-Doppelbindung im Molekül enthalten. Dehydrierte Kohlenwasserstoffe sollen aber auch Kohlenwasserstoffverbindungen umfassen, die ausgehend von Alkylaromaten wie Ethylbenzol oder Isopropylbenzol durch Dehydrierung des Alkylsubstituenten erhältlich sind. Das sind z. B. Verbindungen wie Styrol oder α-Methylstyrol.

Ganz generell bilden dehydrierte Kohlenwasserstoffe wertvolle Ausgangsverbindungen zur Synthese von z. B. funktionalisierten, radikalisch polymerisierbaren Verbindungen (z. B. Acrylsäure aus Propen oder Methacrylsäure aus iso-Buten und deren Polymerisationsprodukte). Beispielsweise können solche funktionalisierten Verbindungen durch Partialoxidation von dehydrierten Kohlenwasserstoffen erzeugt werden. Dehydrierte Kohlenwasserstoffe eignen sich aber auch zur Herstellung von Verbindungen wie Methyl-tert.-butyl-ether (Folgeprodukt von iso-Buten, das z. B. als Kraftstoffadditiv zur Erhöhung der Oktanzahl geeignet ist). Dehydrierte Kohlenwasserstoffe können aber auch als solche selbst zur Polymerisation verwendet werden.

Als zu dehydrierende Kohlenwasserstoffe kommen in dieser Schrift insbesondere die acyclischen und cyclischen Alkane, aber auch Olefine (deren C, C-Doppelbindungszahl erhöht werden soll) in Betracht (als Beispiel sei die heterogen katalysierte partielle Dehydrierung von n-Butenen zu Butadien erwähnt).

D.h., der Begriff "zu dehydrierende Kohlenwasserstoffe" umfasst in dieser Patentanmeldung z.B. Kohlenwasserstoffe der Stöchiometrie CₙH₂ₙ₊₂ mit n > 1 bis n ≤ 20, sowie der Stöchiometrie CₙH₂ₙ mit n > 1 bis n ≤ 20, sowie der Stöchiometrie CₙH₂ₙ₋₂ mit n > 2 bis n ≤ 20, und n = ganzzahlig, insbesondere C₂- bis C₁₆-Alkane wie z. B. Ethan, Propan (zu Propylen), n-Butan, iso-Butan (zu iso-Buten), n-Pentan, iso-Pentan, n-Hexan, n-Heptan, n-Octan, n-Nonan, n-Decan, n-Undecan, n-Dodecan, n-Tridecan, n-Tetradecan, n-Pentadecan und n-Hexadecan.

Insbesondere aber gelten alle in dieser Schrift getroffenen Aussagen für C₂- bis C₈-Alkane als zu dehydrierende Kohlenwasserstoffe und ganz besonders für C₂-bis C₄-Kohlenwasserstoffe (insbesondere Alkane). D.h., zu dehydrierende Kohlenwasserstoffe sind in dieser Schrift vor allem Ethan, Propan, n-Butan und iso-Butan, aber auch 1-Buten und 2-Buten.

Das Eingangs beschriebene Verfahren zur Herstellung von dehydrierten Kohlenwasserstoffen ist allgemein bekannt (vgl. z. B. WO 03/076370, DE-A 10 2004 032 129, EP-A 731 077, WO 01/96271, WO 01/96270, DE-A 103 16 039, WO 03/011804, WO 00/10961, EP-A 799 169 und DE-A 102 45 585).

Prinzipiell werden die Verfahren zur Herstellung von dehydrierten Kohlenwasserstoffen durch heterogen katalysierte partielle Dehydrierung wenigstens eines zu dehydrierenden Kohlenwasserstoffs in zwei Gruppen unterschieden: oxidative und nicht oxidative heterogen katalysierte partielle Dehydrierungen. Im Unterschied zur oxidativen heterogen katalysierten partiellen Dehydrierung erfolgt die nicht oxidative heterogen katalysierte partielle Dehydrierung ohne Mitwirkung von molekularem Sauerstoff. D.h., der dem zu dehydrierenden Kohlenwasserstoff zu entreißende molekulare Wasserstoff wird unmittelbar als molekularer Wasserstoff entrissen und auch nicht in einem Folgeschritt mit molekularem Sauerstoff wenigstens teilweise oxidativ zu Wasser oxidiert. Die Wärmetönung einer nicht oxidativen Dehydrierung ist somit in jedem Fall endotherm. Bei der oxidativen heterogen katalysierten partiellen Dehydrierung wird der dem zu dehydrierenden Kohlenwasserstoff zu entreißende molekulare Wasserstoff hingegen unter Mitwirkung von molekularem Sauerstoff entrissen. Dieses Entreißen kann unmittelbar als Wasser (H₂O) erfolgen (in diesem Fall spricht man verkürzt auch von einer heterogen katalysierten Oxidehydrierung; ihre Wärmetönung ist in jedem Fall exotherm). Es kann aber auch zunächst als molekularer Wasserstoff erfolgen (d.h. nicht oxidativ bzw. konventionell), der dann in einem Folgeschritt teilweise oder vollständig mit molekularem Sauerstoff zu Wasser (H₂O) oxidiert werden kann (je nach Umfang der Wasserstofffolgeverbrennung kann die Bruttowärmetönung endotherm, exotherm oder neutral sein).

Allen vorgenannten heterogen katalysierten partiellen Dehydrierungen von zu dehydrierenden Kohlenwasserstoffen ist gemein, dass sie bei vergleichsweise hohen Reaktionstemperaturen erfolgen. Typische Reaktionstemperaturen können ≥ 250 °C, häufig ≥ 300 °C, oft ≥ 350 °C, oder ≥ 400 °C, bzw. ≥ 450 °C, oder ≥ 500 °C betragen.

Im Verlauf des Langzeitbetriebs einer kontinuierlich betriebenen heterogen katalysierten partiellen Dehydrierung sind darüber hinaus normalerweise mit der Betriebszeit zunehmend noch höhere Reaktionstemperaturen erforderlich, um den Dehydrierumsatz bei einmaligem Durchgang durch den Reaktionsraum aufrechtzuerhalten. Dies rührt üblicherweise daher, dass die verwendeten Katalysatoren mit zunehmender Betriebszeit in zunehmendem Umfang irreversibel deaktivieren. D.h., selbst dann, wenn man den kontinuierlichen Betrieb immer wieder temporär unterbricht (eine solche Betriebsweise soll vom Begriff "kontinuierlich" in dieser Schrift umfasst sein), um die verwendeten Katalysatoren mittels geeigneter Maßnahmen zu reaktivieren (zu regenerieren), wird mit zunehmender Gesamtbetriebszeit des Verfahrens die Ursprungsaktivität der Katalysatoren mit der Gesamtbetriebszeit zunehmend nicht mehr erreicht. Durch entsprechende Erhöhung der Reaktionstemperatur kann diesem Sachverhalt gegengesteuert werden.

Nachteilig an solchermaßen hohen Reaktionstemperaturen ist, dass relativ zur gewünschten Zielreaktion (zu dehydrierender Kohlenwasserstoff → dehydrierter Kohlenwasserstoff) in mit der Reaktionstemperatur in der Regel zunehmendem Umfang unerwünschte Nebenreaktionen zunehmend an Gewicht gewinnen. Eine dieser unerwünschten Nebenreaktionen ist z. B. die thermische Zersetzung des zu dehydrierenden und/oder des dehydrierten Kohlenwasserstoff zu üblicherweise eine geringere Anzahl an Kohlenstoffatomen aufweisenden Kohlenwasserstoffen.

Als Werkstoff für die Einhüllende bei eingangs beschriebenen Verfahren werden im Stand der Technik verschiedene Stähle empfohlen. Die WO 03/076370 empfiehlt beispielsweise in ihren Ausführungsbeispielen als Werkstoff für die Einhüllende Stahl, der auf der den Reaktionsraum berührenden Seite der Einhüllenden alonisiert, alitiert und/oder aluminiert (d. h., mit Aluminium, bzw. mit Aluminiumoxid, bzw. mit Aluminium und Aluminiumoxid beschichtet) ist. Weitere die Zusammensetzung des Stahls betreffende Angaben macht die WO 03/076370 nicht. Die gleiche Empfehlung macht die WO 03/011804. Als mögliche Alternative empfiehlt sie zusätzlich die Mitverwendung von Sulfiden im Ausgangsgasstrom, zum Zweck der Passivierung der den Reaktionsraum berührenden Seite der Einhüllenden.

Nachteilig an einer Alonisierung bzw. Alitierung und/oder Aluminierung ist jedoch, dass sie im großtechnischen Maßstab nur mit besonderem Aufwand durchgeführt werden kann. Nachteilig an einer Mitverwendung von Sulfiden im Ausgangsgasstrom ist zum einen deren Bedarf und zum anderen, dass sich eine solche Mitverwendung in der Regel negativ auf die Standzeit der für die heterogen katalysierte partielle Dehydrierung verwendeten Katalysatoren und/oder negativ auf die Standzeit der für eine sich gegebenenfalls anschließende heterogen katalysierte partielle Oxidation des dehydrierten Kohlenwasserstoff verwendeten Katalysatoren auswirkt.

Die vorgenannten Nachteile nicht aufweisend ist die Lehre der DE-A 10 2004 032 129, die als Werkstoff für die Einhüllende in ihrem Vergleichsbeispiel unbeschichteten Edelstahl der DIN Werkstoffnummer 1.4841 empfiehlt. Die DE-A 10 2005 051 401 und die DE-A 102005052917 empfehlen in ähnlicher Weise Si-haltigen Edelstahl oder ganz allgemein Stahl, z. B. solchen vom DIN Typ 1.4841, als Werkstoff für die Einhüllende.

Eigene Untersuchungen haben jedoch in überraschender Weise ergeben, dass Stahl als Werkstoff für die den Reaktionsraum berührende Seite der Einhüllenden die thermische Zersetzung von zu dehydrierenden und/oder von dehydrierten Kohlenwasserstoffen zu katalysieren vermag, wodurch sich diese bereits bei vergleichsweise niedrigen Reaktionstemperaturen in unerwünschter Weise bemerkbar macht. Dies gilt nicht zuletzt für den Edelstahl der DIN Werkstoffnummer 1.4841, der nachfolgende Elementzusammensetzung aufweisen darf:

| | |
|---|---|
| 24 bis 26 Gew.-% | Cr, |
| 19 bis 22 Gew.-% | Ni, |
| 1,5 bis 2,5 Gew.-% | Si, |
| ≥ 0 bis ≤ 0,11 Gew.-% | N, |
| ≥ 0 bis ≤ 0,2 Gew.-% | C, |
| ≥ 0 bis 2 Gew.-% | Mn, |
| ≥ 0 bis 0,045 Gew.-% | P, |
| ≥ 0 bis 0,015 Gew.-% | S, |
| | |
| | |
| | |

und im übrigen Fe und herstellungsbedingte Verunreinigungen, wobei die Prozentangaben jeweils auf das Gesamtgewicht bezogen sind.

Im Normalfall beträgt der Stickstoffgehalt dieses Stahltyps signifikant weniger als 0,11 Gew.-% und wird seitens der Stahlproduzenten aus diesem Grund in der Regel nicht spezifiziert.

Im Rahmen der eigenen Untersuchungen wurde ferner gefunden, dass die katalytische Wirkung eines Stahls auf die thermische Zersetzung von zu dehydrierenden und/oder dehydrierten Kohlenwasserstoffen von der Elementzusammensetzung des verwendeten Stahls abhängig ist.

Nachteilig an einer thermischen Zersetzung von zu dehydrierendem und/oder dehydriertem Kohlenwasserstoff ist nicht nur, dass eine solche thermische Zersetzung die Zielproduktselektivität mindert, sondern auch, dass die resultierenden Zersetzungsprodukte bei erhöhter Temperatur alle potentielle Bildner von elementarem Kohlenstoff (Koks) sind, der sich auf der Oberfläche der für die heterogen katalysierte partielle Dehydrierung verwendeten Katalysatoren abzuscheiden vermag, um diesen dadurch beschleunigt und wenigstens teilweise auch irreversibel zu deaktivieren. Zusätzlich verläuft eine thermische Zersetzung von Kohlenwasserstoffen grundsätzlich endotherm und entzieht der eigentlichen Zielreaktion Wärme.

Die Aufgabe der vorliegenden Erfindung bestand daher darin, ein wie eingangs beschriebenes Verfahren unter Verwendung eines für die Oberfläche der Einhüllenden auf ihrer den Reaktionsraum berührenden Seite geeigneten Stahls zur Verfügung zu stellen, der bereits in nicht alonisierter Form sowie auch in Abwesenheit von Sulfiden im Ausgangsgasstrom die thermische Zersetzung von zu dehydrierendem und/oder dehydriertem Kohlenwasserstoff weniger ausgeprägt katalysiert als Edelstahl der DIN Werkstoffnummer 1.4841.

Demgemäss wurde ein Verfahren der kontinuierlichen heterogen katalysierten partiellen Dehydrierung wenigstens eines zu dehydrierenden Kohlenwasserstoffs in der Gasphase, umfassend eine Verfahrensweise, bei der man einen Reaktionsraum, der von einer den Reaktionsraum berührenden (d.h. das Reaktionsgas, d.h. den zu dehydrierenden und den dehydrierten Kohlenwasserstoff berührenden) (materiellen) Einhüllenden umschlossen ist, die wenigstens eine erste Öffnung zur Zufuhr wenigstens eines Ausgangsgasstroms in den Reaktionsraum und wenigstens eine zweite Öffnung zur Entnahme wenigstens eines Produktgasstroms aus dem Reaktionsraum aufweist,
- wenigstens einen, wenigstens einen zu dehydrierenden Kohlenwasserstoff enthaltenden, Ausgangsgasstrom kontinuierlich zugeführt,
- im Reaktionsraum den wenigstens einen zu dehydrierenden Kohlenwasserstoff durch wenigstens ein im Reaktionsraum befindliches Katalysatorbett führt, und unter Erzeugung eines den wenigstens einen dehydrierten Kohlenwasserstoff, nicht umgesetzten zu dehydrierenden Kohlenwasserstoff sowie molekularen Wasserstoff und/oder Wasserdampf enthaltenden Produktgases oxidativ oder nicht oxidativ zu wenigstens einem dehydrierten Kohlenwasserstoff partiell dehydriert, und
- dem Reaktionsraum wenigstens einen Produktgasstrom kontinuierlich entnimmt, gefunden,
das dadurch gekennzeichnet ist, dass
die Oberfläche der Einhüllenden auf ihrer den Reaktionsraum berührenden Seite wenigstens teilweise in einer Schichtdicke d von wenigstens 1 mm (jeweils senkrecht zur an den jeweiligen Berührpunkt auf der den Reaktionsraum berührenden Seite angelegten Tangente vom Reaktionsraum weggerichtet) aus einem Stahl S gefertigt ist, der die nachfolgende Elementzusammensetzung aufweist:

| | |
|---|---|
| 18 bis 30 Gew.-% | Cr, |
| 9 bis 36 Gew.-% | Ni, |
| 1 bis 3 Gew.-% | Si, |
| 0,1 bis 0,3 Gew.-% | N, |
| ≥ 0, bevorzugt 0,03 bis 0,15 Gew.-% | C, |
| ≥ 0 bis 4 Gew.-% | Mn, |
| ≥ 0 bis 4 Gew.-% | Al, |
| ≥ 0 bis 0,05 Gew.-% | P, |
| ≥ 0 bis 0,05 Gew.-% | S, und |
| ≥ 0 bis 0,1, vorzugsweise > 0 bis 0,1, und besonders bevorzugt 0,03 bis 0,08 Gew.-% | eines oder mehrere seltene Erdmetalle, und im übrigen Fe und herstellungsbedingte Verunreinigungen, wobei die Prozentangaben jeweils auf das Gesamtgewicht (des Stahls) bezogen sind. |

Ganz generell ist es im Rahmen der vorstehenden Gehalte bevorzugt, wenn der Gehalt an N ≥ 0,11, besser ≥ 0,12, bevorzugt ≥ 0,13, besonders bevorzugt ≥ 0,14 und noch besser ≥ 0,15 Gew.-% beträgt. Besonders bevorzugt liegt der N-Gehalt bei 0,15 bis 0,18 Gew.-%. Besonders bevorzugt werden die vorstehend als bevorzugt genannten Bereiche kombiniert angewendet.

Erfindungsgemäß vorteilhaft beträgt die Gesamtmenge der herstellungsbedingten Verunreinigungen ganz generell und in gleicher Weise bezogen ≤ 1 Gew.-%, vorzugsweise ≤ 0,75 Gew.-%, besonders bevorzugt ≤ 0,5 Gew.-% und ganz besonders bevorzugt ≤ 0,25 Gew.-%. In der Regel wird die Gesamtmenge der herstellungsbedingten Verunreinigungen des Stahls jedoch ≥ 0,1 Gew.-% betragen. Im Unterschied zu den herstellungsbedingten Verunreinigungen handelt es sich bei den anderen Bestandteilen des Stahls um dessen Eigenschaften bestimmende Legierungsbestandteile. Dies gilt insbesondere für die Elemente Cr, Ni, Si, N und C.

Die seltenen Erdmetalle umfassen die Elemente Cer (Ce), Praseodym (Pr), Neodym (Nd), Promethium (Pm), Samarium (Sm), Europium (Eu), Gadolinium (Gd), Terbium (Tb), Dysprosium (Dy), Holmium (Ho), Erbium (Er), Thulium (Tm), Ytterbium (Yb) und Lutetium (Lu). Erfindungsgemäß bevorzugtes seltenes Erdmetall ist Ce. Erfindungsgemäß bevorzugt weist der relevante Stahl S daher ≥ 0 bis 0,1 Gew.-% Ce oder Ce und eines oder mehrere von Ce verschiedene (insbesondere La, Nd und/oder Pr) seltene Erdmetalle auf.

Erfindungsgemäß vorteilhaft ist die Oberfläche der Einhüllenden (der den Reaktionsraum umgebenden Wand) auf ihrer den Reaktionsraum berührenden Seite zu wenigstens 10 %, vorzugsweise zu wenigstens 20 %, oder zu wenigstens 30 %, ganz besonders bevorzugt zu wenigstens 40 %, oder zu wenigstens 50 %, noch vorteilhafter zu wenigstens 60 % oder zu wenigstens 70 %, besser zu wenigstens 80 % oder zu wenigstens 90 % und am besten zu wenigstens 95 % oder zu wenigstens 100 % ihrer Gesamtfläche in einer Schichtdicke d von wenigstens 1 mm aus dem relevanten Stahl gefertigt. Erfindungsgemäß vorteilhaft beträgt vorgenanntes d wenigstens 2 mm, oder wenigstens 3 mm, oder wenigstens 4 mm, oder wenigstens 5 mm. Selbstverständlich kann d aber auch ≥ 5 mm bis 30 mm, oder bis zu 25 mm, oder bis zu 20 mm, oder bis zu 15 mm, oder bis zu 10 mm betragen. Ganz besonders bevorzugt ist beim erfindungsgemäßen Verfahren die den Reaktionsraum umschließende Einhüllende in ihrer Gesamtheit bzw. zu wenigstens 80 % ihres Gewichtes, besser zu wenigstens 90 % oder zu wenigstens 95 % ihres Gewichtes aus dem erfindungsgemäßen Stahl gefertigt. Erfindungsgemäß bevorzugt sind auch die den wenigstens einen Ausgangsgasstrom zum Reaktionsraum hin- sowie den wenigstens einen Produktgasstrom vom Reaktionsraum wegführenden (Rohr) Leitungen aus dem erfindungsgemäßen Stahl S gefertigt bzw. wenigstens auf ihrer gasberührenden Seite mit solchem Stahl beschichtet. Selbstredend können diese (Rohr) Leitungen (insbesondere die wegführenden) aber auch aus anderen Stählen, z. B. der DIN Werkstoffnummern 1.4910, oder 1.4941, oder 1.4541 oder 1.4841 gefertigt sein. Die Verwendung der erfindungsgemäßen Stähle S ist insbesondere dann von erhöhter Relevanz, wenn Reaktionsgas mit Werkstoff in Berührung kommt, der eine Temperatur ≥ 450°C aufweist.

Die vorgenannten (Rohr) Leitungen werden für einen sachgerechten und sicheren Betrieb bevorzugt mit Einrichtungen zur Kompensation von Längenausdehnungseffekten, wie sie z. B. aufgrund von Temperaturänderungen auftreten können, ausgerüstet, wobei mit Vorteil Kompensatoren eingesetzt werden, die sich durch laterale Wirkungsweise auszeichnen.

Diese in der Regel mehrschichtig ausgeführten Kompensatoren können aus dem gleichen Werkstoff wie die Rohrleitung selbst gefertigt sein. Besonders vorteilhaft sind jedoch Ausführungsformen mit (allgemein: gasdurchlässigem starrem Innenrohr und gasundurchlässiger elastischer Außenhülle (gasundurchlässigem elastischem Außenrohr)) einem das zu führende Gas berührenden inneren, vorzugsweise aus einem erfindungsgemäßen Stahl S gefertigten, Rohrteil, der zweckmäßig eine gasdurchlässige Dehnfuge aufweist, und einem außenliegenden, gasundurchlässigen, elastischen, gewellten Teil, der wenigstens teilweise aus einem insbesondere mechanisch und temperaturbelastbaren Werkstoff gefertigt ist, wie z.B. dem Werkstoff 1.4876 (Bezeichnung nach VdTÜV-Wb 434) bzw. 1.4958/1.4959 (Bezeichnung nach DIN 17459) bzw. IN-COLOY^{®} 800 H bzw. 800 HT oder Nickelbasiswerkstoff 2.4816 (alternative Bezeichnung Alloy 600) oder 2.4851 (alternative Bezeichnung Alloy 601).

Erfindungsgemäß bevorzugt weist der erfindungsgemäß relevante Stahl S folgende Elementzusammensetzung auf:

| | |
|---|---|
| 18 bis 26 Gew.-% | Cr |
| 9 bis 36 Gew.-% | Ni, |
| 1 bis 2,5 Gew.-% | Si, |
| 0,1 bis 0,3 Gew.-% | N, |
| ≥ 0, bevorzugt (unabhängig von den anderen Gehalten) 0,03 bis 0,15 Gew.-% | C, |
| ≥ 0 bis 3 Gew.-% | Mn, |
| ≥ 0 bis 4 Gew.-% | Al, |
| ≥ 0 bis 0,05 Gew.-% | P, |
| ≥ 0 bis 0,05 Gew.-% | S, und |
| ≥ 0 bis 0,1, vorzugsweise > 0 bis 0,1 und besonders bevorzugt 0,03 bis 0,08 (jeweils unabhängig von allen anderen Gehalten) Gew.-% | eines oder mehrere seltene Erdmetalle (vorzugsweise Ce oder Ce und eines oder mehrere andere seltene Erdmetalle) und im übrigen Fe und herstellungsbedingte Verunreinigungen, wobei die Prozentangaben jeweils auf das Gesamtgewicht (des Stahls) bezogen sind. |

Vorteilhafter weist der erfindungsgemäß relevante Stahl S folgende Elementzusammensetzung auf:

| | |
|---|---|
| 20 bis 25 Gew.-% | Cr, |
| 9 bis 20 bzw. (unabhängig von den anderen Gehalten) vorzugsweise bis 15 Gew.-% | Ni, |
| 1,4 bis 2,5 Gew.-% | Si, |
| 0,1 bis 0,3 Gew.-% | N, |
| ≥ 0, bevorzugt (unabhängig von den anderen Gehalten) 0,03 bis 0,15 Gew.-% | C, |
| ≥ 0 bis 3 Gew.-% | Mn, |
| ≥ 0 bis 4 Gew.-% | Al, |
| ≥ 0 bis 0,05 Gew.-% | P, |
| ≥ 0 bis 0,05 Gew.-% | S, und |
| ≥ 0 bis 0,1, vorzugsweise > 0 bis 0,1 und besonders bevorzugt 0,03 bis 0,08 (jeweils unabhängig von allen anderen Gehalten) Gew.-% | eines oder mehrere seltene Erdmetalle (vorzugsweise Ce oder Ce und eines oder mehrere andere seltene Erdmetalle) und im übrigen Fe und herstellungsbedingte Verunreinigungen, wobei die Prozentangaben jeweils auf das Gesamtgewicht (des Stahls) bezogen sind. |

Besonders bevorzugt weist der erfindungsgemäß relevante Stahl S folgende Elementzusammensetzung auf:

| | |
|---|---|
| 20 bis 22 Gew.-% | Cr, |
| 10 bis 12 Gew.-% | Ni, |
| 1,4 bis 2,5 Gew.-% | Si, |
| 0,12 bis 0,2 Gew.-% | N, |
| ≥ 0, bevorzugt (unabhängig von allen anderen Gehalten) 0,05 bis 0,12 Gew.-% | C, |
| ≥ 0 bis 1 Gew.-% | Mn, |
| ≥ 0 bis 2, vorzugsweise (unabhängig von allen anderen Gehalten) 0 Gew.-% | Al, |
| ≥ 0 bis 0,045 Gew.-% | P, |
| ≥ 0 bis 0,015 Gew.-% | S, und |
| ≥ 0 bis 0,1, vorzugsweise > 0 bis 0,1 und besonders bevorzugt 0,03 bis 0,08 (jeweils unabhängig von allen anderen Gehalten) Gew.-% | eines oder mehrere seltene Erdmetalle (vorzugsweise Ce oder Ce und eines oder mehrere andere seltene Erdmetalle) und im übrigen Fe und herstellungsbedingte Verunreinigungen, wobei die Prozentangaben jeweils auf das Gesamtgewicht (des Stahls) bezogen sind. |

Ganz generell ist es im Rahmen aller vorstehend als vorteilhaft bzw. bevorzugt genannten Zusammensetzungen günstig, wenn der Gehalt an N ≥ 0,11, besser ≥ 0,12, bevorzugt ≥ 0,13, besonders bevorzugt ≥ 0,14 und noch besser ≥ 0,15 Gew.-% beträgt. Besonders bevorzugt ist in allen Fällen ein N-Gehalt von 0,15 bis 0,18 Gew.-%. Mit Vorteil werden alle jeweils als bevorzugt genannten Bereiche kombiniert angewendet.

Erfindungsgemäß wesentlich ist, dass insbesondere die bevorzugten Stahlzusammensetzungen S bereits so gewählt sind, dass sie sich nicht nur im Bezug auf die unerwünschte katalytische Wirkung des relevanten Stahls als vorteilhaft erweisen.

Vielmehr berücksichtigt vor allem die getroffene Auswahl der Vorzugsbereiche zusätzlich, dass der als Werkstoff ausgewählte Stahl im Produktionsprozess sich zusätzlich auch hinsichtlich seiner mechanischen Beanspruchbarkeit sowie hinsichtlich seiner Korrosions- und sonstigen Verschleißbeanspruchung als möglichst vorteilhaft erweist. Dieser Sachverhalt ist insbesondere dann von Bedeutung, wenn die Einhüllende im wesentlichen vollständig aus dem gewählten Stahl S gefertigt wird.

Beispielsweise sollte der verwendete Stahl S auch eine geringe Versprödungsneigung unter den für die heterogen katalysierte partielle Dehydrierung anzuwendenden Prozessbedingungen aufweisen. Dies insbesondere bei den beim erfindungsgemäßen Verfahren normalerweise anzuwendenden erhöhten Reaktionstemperaturen. Dies vor allem dann, wenn die Reaktionstemperaturen und/oder Katalysatorregeneriertemperaturen ≥ 400 °C bis ≤ 900 °C, bzw. ≥ 500 °C bis ≤ 800 °C, bzw. ≥ 550 °C bis ≤ 750 °C, bzw. ≥ 600 °C bis ≤ 700 °C betragen. Dabei ist in vorgenannter Forderung auch bereits berücksichtigt, dass das erfindungsgemäße Verfahren der heterogen katalysierten partiellen Dehydrierung vorzugsweise bei Arbeitsdrucken oberhalb von 1 atm durchgeführt wird. D.h., der erfindungsgemäß zu verwendende Stahl S sollte auch als Druckbehälterwerkstoff geeignet sein. Des weiteren sollten die Zeitstandfestigkeit und Warmfestigkeitskennwerte des erfindungsgemäß erwählten Stahls unter den Bedingungen des erfindungsgemäßen Verfahrens befriedigen. Das gleiche trifft auf seine Zunderbeständigkeit und auf seine Aufkohlungsneigung zu. Die getroffene Vorteilsauswahl berücksichtigt ferner, dass der ausgewählte Stahl in vorteilhafter Weise verarbeitbar sein muss. Dies heißt insbesondere, dass er schweißtechnisch verarbeitbar sein muss, was bei vorab der Verarbeitung alonisierten Stählen ohne Beeinträchtigung der Alonisierung nicht möglich ist. Eine Alonisierung nach der Verarbeitung ist jedoch im großtechnischen Maßstab nur sehr aufwendig bzw. gar nicht durchführbar. Das Erfordernis der Korrosionsbeständigkeit ist insbesondere auch unter dem Gesichtspunkt von Bedeutung, dass sich der beim erfindungsgemäßen Verfahren gebildete dehydrierte Kohlenwasserstoff in einem sich an das erfindungsgemäße Verfahren anschließenden Verfahren (besonders vorteilhaft in Begleitung des nicht dehydrierten Kohlenwasserstoffs) heterogen katalysiert partialoxidieren lässt. Trennt man nachfolgend aus dem Produktgasgemisch der Partialoxidation das gewünschte Partialoxidationsprodukt ab, verbleibt normalerweise ein noch nicht dehydrierten Kohlenwasserstoff und Oxygenate enthaltendes Restgas, das vorteilhaft zum Zweck der weiteren Umsetzung des noch nicht dehydrierten Kohlenwasserstoffs wenigstens teilweise in das erfindungsgemäße Verfahren rückgeführt wird. Der überwiegenden Mehrzahl aller Oxygenate (z.B. Acrolein, Acrylsäure, Methacrolein, Methacrylsäure, H₂O, O₂, CO₂ etc.) kommt jedoch korrosive Wirkung zu.

Die erfindungsgemäß geeigneten Stähle S sind in an sich bekannter Weise herstellbar. Vgl. z.B. die Ausführungen in Enzyklopädie Naturwissenschaft und Technik, Verlag moderne Industrie, 1976, unter den Stichworten "Stahlbegleiter", "Eisen" sowie "Eisen und Stahl", oder Ullmanns Encyclopädie der technischen Chemie, Verlag Chemie, 4. Auflage, Band 3, Verfahrenstechnik II und Reaktionsapparate, Kapitel "Werkstoffe in der chemischen Industrie".

Erfindungsgemäß geeignete Stähle sind z.B. die Stähle der EN Werkstoffnummern 1.4818, 1.4835 und 1.4854, unter denen jener der Werkstoffnummer 1.4835 ganz besonders bevorzugt ist. Ferner eignen sich für das erfindungsgemäße Verfahren auch die Stähle der DIN Werkstoffnummern 1.4891 und 1.4893, von denen derjenige mit der Werkstoffnummer 1.4893 bevorzugt ist. Auch eignen sich für das erfindungsgemäße Verfahren Stähle der ASTM/UNS Werkstoffnummern S 30415, S 30815 und S 35315, innerhalb derer derjenige mit der Werkstoffnummer S 30815 erfindungsgemäß bevorzugt wird. Außerdem eignen sich für das erfindungsgemäße Verfahren Stähle mit den SS Werkstoffnummern 2372, 2361 und 2368, unter welchen der letztere erfindungsgemäß bevorzugt wird.

Vorgenannte und sonstige erfindungsgemäß geeignete Stähle S sind auch im Handel erhältlich. Beispielsweise vertreibt die Firma Outokumpu Stainless AB, in SE-774 22 Avesta, Schweden unter den firmeneigenen Bezeichnungen 153 MA™, 253 MA^{®} und 353 MA^{®} von den vorgenannten Stählen, unter denen der 253 MA^{®} erfindungsgemäß besonders vorteilhaft ist. Die Firma ThyssenKrupp Nirosta GmbH, in D-47794 Krefeld, Germany, vertreibt mit dem legierten Edelstahl THERMAX^{®} 4835 gleichfalls einen erfindungsgemäß besonders günstigen Stahl.

Zu den erfindungsgemäß geeigneten Stählen S gehören somit insbesondere Stähle der Zusammensetzungen:
I.

| | |
|---|---|
| 18,5 Gew.-% | Cr, |
| 9,5 Gew.-% | Ni, |
| 1,3 Gew.-% | Si, |
| 0,15 Gew.-% | N, |
| ≥ 0 bis ≤ 0,05 Gew.-% | C, |
| ≥ 0 bis ≤ 1 Gew.-% | Mn, |
| ≥ 0 bis ≤ 0,045 Gew.-% | P, |
| ≥ 0 bis ≤ 0,015 Gew.-% | S, und |
| ≥ 0 bis 0,1, vorzugsweise > 0 bis 0,1 und besonders bevorzugt 0,03 bis 0,08 Gew.-% | eines oder mehrere seltene Erdmetalle (vorzugsweise Ce oder Ce und eines oder mehrere andere seltene Erdmetalle), und im übrigen Fe und herstellungsbedingte Verunreinigungen, wobei die Prozentangaben jeweils auf das Gesamtgewicht bezogen sind. |

II.

| | |
|---|---|
| 21 Gew.-% | Cr, |
| 11 Gew.-% | Ni, |
| 1,4 bis 2,5 Gew.-% | Si, |
| 0,17 Gew.-% | N, |
| 0,09 Gew.-% | C, |
| ≥ 0 bis ≤ 1 Gew.-% | Mn, |
| ≥ 0 bis ≤ 0,045 Gew.-% | P, |
| ≥ 0 bis ≤ 0,015 Gew.-% | S und |
| ≥ 0 bis 0,1, vorzugsweise > 0 bis 0,1 und besonders bevorzugt 0,03 bis 0,08 Gew.-% | eines oder mehrere seltene Erdmetalle (vorzugsweise Ce oder Ce und eines oder mehrere andere seltene Erdmetalle), und im übrigen Fe und herstellungsbedingte Verunreinigungen, wobei die Prozentangaben jeweils auf das Gesamtgewicht bezogen sind. |

III.

| | |
|---|---|
| 24 bis 26 Gew.-% | Cr, |
| 34 bis 36 Gew.-% | Ni, |
| 1,2 bis 2,0 Gew.-% | Si, |
| 0,12 bis 0,2 Gew.-% | N, |
| 0,04 bis 0,08 Gew.-% | C, |
| ≥ 0 bis ≤ 2,0 Gew.-% | Mn, |
| ≥ 0 bis ≤ 0,045 Gew.-% | P, |
| ≥ 0 bis ≤ 0,015 Gew.-% | S und |
| ≥ 0 bis 0,1, vorzugsweise > 0 bis 0,1 und besonders bevorzugt 0,03 bis 0,08 Gew.-% | eines oder mehrere seltene Erdmetalle (vorzugsweise Ce oder Ce und eines oder mehrere andere seltene Erdmetalle), und im übrigen Fe und herstellungsbedingte Verunreinigungen, wobei die Prozentangaben jeweils auf das Gesamtgewicht bezogen sind. |

Selbstredend können die erfindungsgemäß geeigneten Stähle S auch in alonisierter, alitierter und/oder aluminierter Form (insbesondere auf ihrer den Reaktionsraum berührenden Seite) für das erfindungsgemäße Verfahren verwendet werden. Sie erweisen sich auch in dieser Form insofern als besonders vorteilhaft, als dort, wo die Alonisierung z.B. fertigungsbedingt beschädigt ist oder wird, nur eine die thermische Zersetzung geringfügig katalysierende Wirkung zur Geltung kommt.

Soll die Einhüllende beim erfindungsgemäßen Verfahren nicht vollständig aus erfindungsgemäß geeignetem Stahl gefertigt sondern nur teilweise oder vollständig die Oberfläche der Einhüllenden auf ihrer den Reaktionsraum berührenden Seite in erfindungsgemäßer Mindestschichtdicke aus solchem Stahl gefertigt sein (beispielsweise kann es aus Gründen der Wirtschaftlichkeit zweckmäßig sein, die Oberfläche der Einhüllenden auf ihrer den Reaktionsraum berührenden Seite nur dort erfindungsgemäß zu fertigen, wo im Reaktionsraum besonders hohe Temperaturen auftreten oder überhaupt nur die Oberfläche in erfindungsgemäßer Mindestschichtdicke aus erfindungsgemäßem Stahl zu fertigen), ist dies in einfacher Weise z.B. durch Plattieren (z.B. Walzplattieren, oder Sprengplattieren, oder Pressschweißplattieren) möglich, d.h., dadurch, dass man auf die Oberfläche eines anderen Grundwerkstoffs (z.B. eines anderen Stahls) an den gewünschten Stellen oder im Gesamtumfang der Oberfläche den erfindungsgemäßen Stahl als Deckwerkstoff aufbringt und z.B. durch Pressen, oder Walzen, bzw. Verkleben oder Verschweißen fest mit dem Grundwerkstoff verbindet.

Das erfindungsgemäße Verfahren ist insbesondere dann vorteilhaft, wenn der wenigstens eine Ausgangsgasstrom als inertes Verdünnungsgas Wasserdampf (z.B. ≥ 1 Vol.-%) und/oder als Reaktand molekularen Sauerstoff (z.B. ≥ 0,1 oder ≥ 0,5 Vol.-%) enthält. Es ist aber auch dann vorteilhaft, wenn der wenigstens eine Ausgangsgasstrom Wasserdampf und/oder molekularen Sauerstoff als Verunreinigungen enthält. Es ist ferner dann von Vorteil, wenn im Verlauf der erfindungsgemäßen heterogen katalysierten partiellen Dehydrierung Wasserdampf als Reaktionsprodukt gebildet wird. Dies insbesondere dann, wenn für das erfindungsgemäße Verfahren die in der WO 03/076370 empfohlene Kreisgas- bzw. Schlaufenfahrweise angewendet wird. Das erfindungsgemäße Verfahren ist aber nicht zuletzt dann besonders vorteilhaft, wenn der wenigstens eine Ausgangsgasstrom aus einer dem erfindungsgemäßen Verfahren nachgeschalteten Partialoxidation von im Rahmen des erfindungsgemäßen Verfahrens gebildetem dehydriertem Kohlenwasserstoff in Begleitung von noch zu dehydrierendem Kohlenwasserstoff stammendes, nach Zielproduktabtrennung aus dem Produktgasgemisch der Partialoxidation verbliebenes, Oxygenat enthaltendes Restgas umfasst.

Ganz generell kann das wenigstens eine im Reaktionsraum befindliche Katalysatorbett sowohl ein Wirbelbett, oder ein Fließbett, oder ein Festbett sein. Selbstredend können Wirbelbett und z.B. Festbett, oder Fließbett und Festbett im Reaktionsraum auch kombiniert enthalten sein. Erfindungsgemäß bevorzugt handelt es sich bei dem wenigstens einen Katalysatorbett des erfindungsgemäßen Verfahrens ausschließlich um Katalysatorfestbetten.

Unter der Belastung eines einen Reaktionsschritt katalysierenden Katalysatorbetts mit Reaktionsgas soll in dieser Schrift ganz generell die Menge an Reaktionsgas in Normlitern (= NI; das Volumen in Litern, das die entsprechende Reaktionsgasmenge bei Normalbedingungen (0°C, 1 atm) einnehmen würde) verstanden, die pro Stunde durch einen Liter Katalysatorbett (z.B. Katalysatorfestbett) geführt wird. Die Belastung kann aber auch nur auf einen Bestandteil des Reaktionsgases bezogen sein. Dann ist es die Menge dieses Bestandteils in NI/I•h, die pro Stunde durch einen Liter des Katalysatorbetts geführt wird (reine Inertmaterialschüttungen werden nicht zu einem Katalysatorfestbett gerechnet). Die Belastung kann auch nur auf die in einem Katalysatorbett, das den eigentlichen Katalysator mit Inertmaterial verdünnt enthält, enthaltene Menge an Katalysator bezogen sein (dies wird dann explizit vermerkt).

Unter einer vollständigen Oxidation (Verbrennung) eines dehydrierten und/oder zu dehydrierenden Kohlenwasserstoffs wird in dieser Schrift verstanden, dass der im Kohlenwasserstoff insgesamt enthaltene Kohlenstoff in Oxide des Kohlenstoffs (CO, CO₂) umgewandelt wird. Alle davon verschiedenen Umsetzungen eines dehydrierten und/oder zu dehydrierenden Kohlenwasserstoffs unter reaktiver Einwirkung von molekularem Sauerstoff werden in dieser Schrift mit dem Begriff der Partialoxidation subsummiert. Die zusätzliche reaktive Einwirkung von Ammoniak kennzeichnet die Ammoxidation, die ebenfalls unter dem Begriff der Partialoxidation subsummieren soll.

Als ein Inertgas soll in dieser Schrift generell ein Reaktionsgasbestandteil verstanden werden, der sich unter den Bedingungen der entsprechenden Reaktion im wesentlichen als chemisch inert verhält und - jeder inerte Reaktionsgasbestandteil für sich betrachtet - zu mehr als 95 mol-%, vorzugsweise zu mehr als 97 mol-% bzw. zu mehr als 99 mol-% chemisch unverändert erhalten bleibt. Beispiele für typische inerte Verdünnungsgase sind z.B. N₂, H₂O, CO₂, Edelgase wie Ne und Ar sowie Mischungen aus diesen Gasen etc..

Handelt es sich beim erfindungsgemäßen Verfahren um eine heterogen katalysierte Oxidehydrierung (z.B. um diejenige von Propan zu Propylen), kann als Quelle des dafür benötigten molekularen Sauerstoff Luft, reiner molekularer Sauerstoff oder an molekularem Sauerstoff angereicherte Luft eingesetzt werden.

Die Notwendigkeit der Durchführung des erfindungsgemäßen Verfahrens an im festen Zustand befindlichen selektiv wirkenden Dehydrierkatalysatoren ist dadurch bedingt, dass die Dehydrierung (Spaltung von C-H) gegenüber der thermischen Spaltung bzw. Crackung (Spaltung von C-C) kinetisch benachteiligt ist. Infolge der selektiv wirkenden Katalysatoren sowie in Anwendung eines erfindungsgemäßen Reaktionsraumes entstehen im Fall einer erfindungsgemäßen heterogen katalysierten Dehydrierung von Propan Nebenprodukte wie Methan, Ethylen und Ethan nur in untergeordneten Mengen.

Unter einem Dehydrierkatalysator soll daher in dieser Schrift im besonderen ein Formkörper verstanden werden, dessen Längstausdehnung L (längste direkte Verbindungslinie zweier auf der Oberfläche des Formkörpers befindlicher Punkte) 0,1 bzw. 1 bis 30 mm, vorzugsweise 1 bis 20 mm und besonders bevorzugt 1 bis 10 mm bzw. 1 bis 5 mm beträgt und der im nachfolgend beschriebenen Versuch, bezogen auf einmaligen Durchgang des Reaktionsgases durch das Reaktionsrohr, wenigstens 5 mol-% des im Reaktionsgas enthaltenen Propan zu Propylen dehydriert:
Ein Reaktionsrohr aus erfindungsgemäß zu verwendendem Stahl der EN Werkstoffnummer 1.4835 mit einer Wanddicke von 2 mm und einem Innendurchmesser von 35 mm sowie einer Länge von 80 cm wird wie folgt befüllt. 50 ml aus einer Schüttung des entsprechenden Dehydrierkatalysator werden im Reaktionsrohr mittig platziert. Oberhalb und unterhalb der Schüttung aus Katalysatorformkörperwird das Reaktionsrohr jeweils mit einer Schüttung aus Steatitkugeln (Inertkugeln) mit einem Kugeldurchmesser von 1,5 mm bis 2,5 mm aufgefüllt. Ein Gitterrost trägt die gesamte Schüttung. Von außen wird das Reaktionsrohr auf seiner gesamten Länge auf einer Temperatur von 550°C gehalten. Das Reaktionsrohr wird mit einem Gemisch aus Propan und Wasserdampf im Volumenverhältnis 2 (Propan) zu 1 (Wasserdampf) mit einer Propanbelastung der Katalysatorschüttung von 1000 NI/I•h beschickt. Der Ausgangsgasstrom ist auf eine Temperatur von 550°C vorerwärmt. Besonders bevorzugt sind Dehydrierkatalysatoren, bei denen unter vorstehenden Rahmenbedingungen die Summenselektivität der Bildung der Nebenprodukte Ethan, Ethylen und Methan ≤ 5 mol-%, bezogen auf umgesetztes Propan, beträgt.

Eine erfindungsgemäße heterogen katalysierte Oxidehydrierung lässt sich prinzipiell so durchführen, wie zum Beispiel in den Schriften US-A 4,788,371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US-A 3,862,256, US-A 3,887,631, DE-A 195 30 454, US-A 4,341,664, J. of Catalysis 167, 560-569 (1997), J. of Catalysis 167, 550-559 (1997), Topics in Catalysis 3 (1996) 265-275, US 5,086,032, Catalysis Letters 10 (1991), 181-192, Ind. Eng. Chem. Res. 1996, 35, 14-18, US 4,255,284, Applied Catalysis A: General, 100 (1993), 111-130, J. of Catalysis 148, 56-67 (1994), V. Cortes Corberän und S. Vic Bellón (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V. , S. 375ff oder in DE-A 198 37 520, DE-A 198 37 517, DE-A 198 37 519 und DE-A 198 37 518 am Beispiel der heterogen katalysierten partiellen Oxidehydrierung von Propan beschrieben. Dabei kann, wie bereits gesagt, als Sauerstoffquelle z.B. Luft eingesetzt werden. Häufig weist die verwendete Sauerstoffquelle neben Inertgas hier jedoch zu mindestens 90 mol.-% molekularen Sauerstoff, und vielfach zu wenigstens 95 mol-% molekularen Sauerstoff auf.

Die für die heterogen katalysierte Oxidehydrierung geeigneten Katalysatoren unterliegen keinen besonderen Beschränkungen. Es eignen sich alle dem Fachmann auf diesem Gebiet bekannten Oxidehydrierungskatalysatoren, die in der Lage sind, z.B. Propan zu Propylen zu oxidieren. Insbesondere können alle in den zuvor genannten Schriften genannten Oxidehydrierungskatalysatoren eingesetzt werden. Geeignete Katalysatoren sind beispielsweise Oxidehydrierungskatalysatoren, die MoVNb-Oxide oder Vanadylpyrophosphat, gegebenenfalls mit Promotor, umfassen. Ein Beispiel für einen günstigen Oxidehydrierkatalysator ist ein Katalysator, der ein Mischmetalloxid I mit Mo, V, Te, O und X als wesentliche Bestandteile enthält, wobei X mindestens ein Element ist, ausgewählt aus Niob, Tantal, Wolfram, Titan, Aluminium, Zirkonium, Chrom, Mangan, Gallium, Eisen, Ruthenium, Kobalt, Rhodium, Nickel , Palladium, Platin, Antimon, Bismut, Bor, Indium, Silizium, Lanthan, Natrium, Lithium, Kalium, Magnesium, Silber, Gold und Cer (vgl. dazu auch EP-A 938463 und EP-A 167109). Weitere besonders geeignete Oxidehydrierungskatalysatoren sind die Multimetalloxidmassen beziehungsweise -katalysatoren A (in dieser Schrift Multimetalloxidmassen II genannt) der DE-A-197 53 817 und die Katalysatoren der DE-A 19838312, wobei die in der erstgenannten Schrift als bevorzugt genannten Multimetalloxidmassen beziehungsweise - katalysatoren A ganz besonders günstig sind. Damit kommen für eine erfindungsgemäß heterogen katalysierte Oxidehydrierung als Aktivmassen u.a. Multimetalloxidmassen der allgemeinen Formel III

M¹ₐMo_{1-b}M²_{b}Oₓ (III),

mit
M¹ = Co, Ni, Mg, Zn, Mn und/oder Cu,
M² = W, V, Te, Nb, P, Cr, Fe, Sb, Ce, Sn und/oder La,
a = 0,5-1,5,
b = 0-0,5,
sowie
x = eine Zahl, die durch die Wertigkeit und Häufigkeit
   der von Sauerstoff verschiedenen Elemente in (III) bestimmt
   wird,

in Betracht. Ihre Herstellung und Formgebung kann wie in der DE-A 102 45 585 beschrieben erfolgen.

Für eine heterogen katalysierte Oxidehydrierung von z.B. Propan liegt die Reaktionstemperatur bei Verwendung frischer Katalysatoren vorzugsweise im Bereich von 200 bis 600°C, insbesondere im Bereich von 250 bis 500°C, stärker bevorzugt im Bereich von 350 bis 440°C. Der Arbeitsdruck liegt vorzugsweise im Bereich von 0,5 bis 10 bar, insbesondere von 1 bis 10 bar, stärker bevorzugt von 1 bis 5 bar. Arbeitsdrucke oberhalb von 1 bar, zum Beispiel von 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren (die Rohrwand bildet zusammen mit den beiden Rohröffnungen die den Reaktionsraum berührende Einhüllende; das Rohrinnere ist der Reaktionsraum; vorzugsweise ist die Rohrwand vollständig aus erfindungsgemäßem Stahl gefertigt) eines z. B. salzbadgekühlten Rohrbündelreaktors aufgeschüttet (in der Regel auf einem gasdurchlässigen Gitterrost befindlich), wie sie zum Beispiel in der EP-A 700 893 und in der EP-A 700 714 sowie der in diesen Schriften zitierten Literatur beschrieben sind. Der Ausgangsgasstrom wird dem Rohreingang zugeführt. Die mittlere Verweilzeit des Reaktionsgases in der Katalysatorschüttung liegt zweckmäßigerweise bei 0,5 bis 20 Sekunden. Das Verhältnis von Propan zu Sauerstoff variiert mit dem gewünschten Umsatz und der Selektivität des Katalysators. Zweckmäßigerweise liegt es im Bereich von 0,5:1 bis 40:1, insbesondere von 1:1 bis 6:1, stärker bevorzugt von 2:1 bis 5:1. In der Regel nimmt die Propylenselektivität mit steigendem Propanumsatz ab. Vorzugsweise wird deshalb die Propan zu Propylen-Reaktion so durchgeführt, dass relativ niedrige Umsätze mit Propan bei hohen Selektivitäten zu Propylen erreicht werden. Besonders bevorzugt liegt der Umsatz an Propan im Bereich von 5 bis 40 mol-%, häufig im Bereich von 10 bis 30 mol-%. Hierbei bedeutet der Begriff "Propanumsatz" den Anteil an zugeführtem Propan, der beim einfachen Durchgang des Reaktionsgases durch das Rohr umgesetzt wird. In der Regel beträgt die Selektivität der Propylenbildung 50 bis 98 mol-%, stärker bevorzugt 80 bis 98 mol-%, wobei der Begriff "Selektivität" die Mole an Propylen bezeichnet, die pro Mol umgesetztem Propan erzeugt werden, ausgedrückt als molarer Prozentsatz. Im Reaktionsrohr durchläuft die Reaktionstemperatur in der Regel ein Maximum.

In der Regel enthält der bei einer heterogen katalysierten Propanoxidehydrierung eingesetzte Ausgangsgasstrom 5 bis 95 mol-% Propan (bezogen auf 100 mol-% Ausgangsgas). Neben Propan und Sauerstoff kann das Ausgangsgas für die heterogen katalysierte Oxidehydrierung auch weitere, insbesondere inerte, Bestandteile wie Kohlendioxid, Kohlenmonoxid, Stickstoff, Edelgase, andere Kohlenwasserstoffe, z.B. im Roh-Propan (als Propanquelle wird für das erfindungsgemäße Verfahren normalerweise Roh-Propan verwendet, wie es z.B. in der DE-A 10245585 bzw. in der DE-A 102005022798 empfohlen wird) enthaltene Nebenbestandteile, und/oder Propylen umfassen. Die heterogen katalysierte Oxidehydrierung kann auch in Anwesenheit von Verdünnungsmitteln, wie zum Beispiel Wasserdampf, durchgeführt werden.

Jede beliebige Reaktorsequenz kann zur Durchführung der heterogen katalysierten Oxidehydrierung von z. B. Propan eingesetzt werden, die dem Fachmann bekannt ist. Zum Beispiel kann die heterogen katalysierte Oxidehydrierung in einem einzigen Reaktor oder in einer Kaskade aus zwei oder mehreren Reaktoren, zwischen denen gegebenenfalls Sauerstoff zugeführt wird, durchgeführt werden.

Als mögliche Bestandteile kann das Produktgas einer erfindungsgemäßen heterogen katalysierten Propandehydrierung zum Beispiel folgende Bestandteile enthalten: Propylen (als Zielprodukt, d.h., als dehydrierter Kohlenwasserstoff), Propan (als nicht umgesetzter zu dehydrierender Kohlenwasserstoff), Kohlendioxid, Kohlenmonoxid, Wasser, Stickstoff, Sauerstoff, Ethan, Ethen, Methan, Acrolein, Acrylsäure, Ethylenoxid, Butan (z.B. n-Butan oder iso-Butan), Essigsäure, Formaldehyd, Ameisensäure, Propylenoxid und Butene (z.B. Buten-1). Dabei bilden insbesondere Ethan, Ethen und Methan mögliche thermische Zersetzungsprodukte von Propan. In typischer Weise enthält ein bei einer erfindungsgemäßen heterogen katalysierten Propanoxidehydrierung erhaltenes Produktgas: 5 bis 10 mol-% Propylen, 0,1 bis 2 mol-% Kohlenmonoxid, 1 bis 3 mol-% Kohlendioxid, 4 bis 10 mol-% Wasser, 0 bis 1 mol-% Stickstoff, 0,1 bis 0,5 mol-% Acrolein, 0 bis 1 mol-% Acrylsäure, 0,05 bis 2 mol-% Essigsäure, 0,01 bis 0,05 mol-% Formaldehyd, 1 bis 5 mol-% Sauerstoff, 0,1 bis 10 mol-% weitere oben genannte Bestandteile, sowie als Rest im wesentlichen Propan, jeweils bezogen auf 100 % Produktgas.

Heterogen katalysierte Oxidehydrierungen von von Propan verschiedenen zu dehydrierenden Kohlenwasserstoffen können erfindungsgemäß in entsprechender Weise durchgeführt werden, wie vorstehend für die Oxidehydrierung von Propan beschrieben. Als solche zu oxidehydrierenden Kohlenwasserstoffe kommen insbesondere Butan (zu Buten (vor allem iso-Butan zu iso-Buten) und/oder Butadien) sowie Butene (zu Butadien) in Betracht.

Die Regenerierung von für eine partielle heterogen katalysierte Oxidehydrierung von z.B. Propan verwendeten Katalysatoren kann so wie für Partialoxidationskatalysatoren in den Schriften DE-A 103 51 269, DE-A 103 50 812 und DE-A 103 50 822 beschrieben vorgenommen werden.

Handelt es sich bei der erfindungsgemäßen heterogen katalysierten partiellen Dehydrierung nicht um eine Oxidehydrierung, so beinhaltet sie stets eine konventionelle heterogen katalysierte Dehydrierung, d.h. es wird wenigstens intermediär molekularer Wasserstoff gebildet, der im Fall einer oxidativen (konventionellen) heterogen katalysierten partiellen Dehydrierung in einem Folgeschritt mit molekularem Sauerstoff wenigstens teilweise zu Wasser verbrannt wird.

Als Katalysatoren für eine heterogen katalysierte konventionelle Dehydrierung von zu dehydrierenden Kohlenwasserstoffen, kommen im Rahmen des erfindungsgemäßen Verfahrens grundsätzlich alle im Stand der Technik für konventionelle heterogen katalysierte Dehydrierungen bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit alle Dehydrierkatalysatoren eingesetzt werden, die in der WO 01/96270, der EP-A 731077, der DE-A 10211275, der DE-A 10131297, der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105, der US-A 3,670,044, der US-A 6,566,573, der US-A 4,788,371, der WO 94/29021 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Generell kann es sich bei den Dehydrierkatalysatoren um Katalysatorstränge (Durchmesser typisch 0,1 bzw. 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln. Für eine heterogen katalysierte Dehydrierung im Wirbelbett (bzw. Fließ- oder Wanderbett) wird man entsprechend feinteiligeren Katalysator einsetzen. Erfindungsgemäß bevorzugt ist das Katalysatorfestbett.

In der Regel sind die Dehydrierkatalysatoren (insbesondere die in der DE-A 199 37107 empfohlenen (insbesondere die beispielhaften Katalysatoren dieser DE-A)) so beschaffen, dass sie sowohl die Dehydrierung des zu dehydrierenden Kohlenwasserstoffs (z.B. Propan) als auch die Verbrennung des zu dehydrierenden Kohlenwasserstoff (z.B. Propan) und von molekularem Wasserstoff zu katalysieren vermögen. Die Wasserstoffverbrennung verläuft dabei sowohl im Vergleich zur Dehydrierung des zu dehydrierenden Kohlenwasserstoff (z.B. Propans) als auch im Vergleich zu dessen Verbrennung im Fall einer Konkurrenzsituation an den Katalysatoren sehr viel schneller.

Ferner kommen für eine erfindungsgemäße konventionelle heterogen katalysierte partielle Dehydrierung eines zu dehydrierenden Kohlenwasserstoffs (z.B. Propan) prinzipiell alle im Stand der Technik bekannten Reaktortypen und Verfahrensvarianten in Betracht, sofern der Reaktionsraum im jeweiligen Reaktor das erfindungsgemäße Anforderungsprofil erfüllt. Beschreibungen solcher Verfahrensvarianten enthalten zum Beispiel alle bezüglich der Dehydrierkatalysatoren zitierten Schriften des Standes der Technik sowie der eingangs dieser Schriften zitierte Stand der Technik.

Eine vergleichsweise ausführliche Beschreibung von auch für erfindungsgemäße Reaktionsräume geeigneten Verfahren der konventionellen heterogen katalysierten Dehydrierung (nicht oxidativ oder oxidativ) enthält z.B. Catalytica® Studies Division, Oxidative Dehydogenation and Alternative DehydrogenationProcesses, Study Number 4192 OD, 1993, 430 Ferguson Drive, Mountain View, California, 94043-5272 U.S.A..

Charakteristisch für die konventionelle partielle heterogen katalysierte Dehydrierung von zu dehydrierenden Kohlenwasserstoffen (z.B. Propan) ist, dass der Dehydrierschritt endotherm verläuft. Das heißt, die für die Einstellung der erforderlichen Reaktionstemperatur benötigte Wärme (Energie) muss entweder dem Reaktionsgas vorab und/oder im Verlauf der heterogen katalysierten Dehydrierung zugeführt werden.

D.h., bezogen auf den einmaligen Durchgang des wenigstens einen Ausgangsgasstroms durch das wenigstens eine Katalysatorbett im erfindungsgemäß beschaffenen Reaktionsraum, kann der Reaktionsraum durch gezielten Wärmeaustausch mit z.B. außerhalb des von der erfindungsgemäßen Einhüllenden umschlossenen Reaktionsraums geführten fluiden (d.h. flüssigen oder gasförmigen) Wärmeträgern isotherm gestaltet werden (extern gelenkter Temperaturverlauf). Entsprechende Wärmeaustauscher können aber auch im Reaktionsraum selbst untergebracht sein.

Sie kann mit gleicher Bezugsbasis aber auch adiabat, d.h. im wesentlichen ohne einen solchen gezielten Wärmeaustausch mit (extern) geführten Wärmeträgern ausgeführt werden (extern ungelenkter Temperaturverlauf). Im letzteren Fall kann die auf den einmaligen Durchgang durch den erfindungsgemäßen Reaktionsraum bezogene Bruttowärmetönung durch im folgenden noch zu beschreibenden intern gelenkten (z.B. durch Wasserstoffverbrennung in einem Folgeschritt) Temperaturverlauf sowohl endotherm (negativ), oder autotherm (im wesentlichen Null) oder exotherm (positiv) gestaltet werden.

In typischer Weise benötigt eine erfindungsgemäße konventionelle heterogen katalysierte partielle Dehydrierung wenigstens eines zu dehydrierenden Kohlenwasserstoffs (z.B. von Propan), wie bereits erwähnt, vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 800°C, bzw. 400 bis 700°C. Pro Molekül an z.B. zu Propylen dehydriertem Propan wird dabei ein Molekül Wasserstoff erzeugt. Hohe Temperaturen und Entfernung des Reaktionsproduktes H₂ begünstigen die Gleichgewichtslage im Sinne des Zielprodukts ebenso wie Partialdruckerniedrigung durch inerte Verdünnung.

Ferner ist es für konventionelle heterogen katalysierte partielle Dehydrierungen wenigstens eines zu dehydrierenden Kohlenwasserstoffs (z.B. von Propan) aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur konventionellen heterogen katalysierten Dehydrierung bei erhöhter Temperatur über die Katalysatoroberfläche zu leitende, den dehydrierenden Kohlenwasserstoff (z.B. das Propan) enthaltende Ausgangsgas mit Wasserdampf verdünnt werden. Sich abscheidender Kohlenstoff wird unter den so gegebenen Bedingungen nach dem Prinzip der Kohlevergasung kontinuierlich teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine gewisse Unterdrückung der Bildung von Kohlenstoffablagerungen ist aber auch dadurch möglich, dass man dem konventionell heterogen katalysiert zu dehydrierenden Kohlenwasserstoff (z. B. Propan), bevor er bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Kohlenwasserstoff (z. B. Propan) Wasserdampf und molekularen Wasserstoff im Gemisch zuzusetzen. Ein Zusatz von molekularem Wasserstoff zur heterogen katalysierten Dehydrierung von Propan mindert auch die unerwünschte Bildung von Allen (Propadien), Propin und Acetylen als Nebenprodukten.

Erfindungsgemäß kann es daher (wie bereits angesprochen) zweckmäßig sein, die konventionelle heterogen katalysierte Kohlenwasserstoff(z. B. Propan)dehydrierung (z.B. mit vergleichsweise geringem Propan(bzw. generell Kohlenwasserstoff)umsatz) (quasi) adiabat durchzuführen. Das heißt, man wird das Ausgangsgas in der Regel zunächst auf eine Temperatur von 400 bzw. 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch wenigstens ein im erfindungsgemäßen Reaktionsraum befindliches Katalysatorbett ausreichend sein, um den gewünschten Umsatz zu erzielen, wobei sich das Reaktionsgas um etwa 30°C bis 200°C (je nach Umsatz und Verdünnung) abkühlen wird. Ein Beisein von Wasserdampf als Wärmeträger macht sich auch unter dem Gesichtspunkt einer adiabaten Fahrweise vorteilhaft bemerkbar. Die vergleichsweise niedrige Reaktionstemperatur ermöglicht längere Standzeiten des verwendeten Katalysatorbetts.

Prinzipiell ist eine erfindungsgemäße konventionelle heterogen katalysierte Kohlenwasserstoff(z. B. Propan)dehydrierung (unabhängig ob adiabat oder isotherm gefahren) sowohl im Katalysatorfestbett, als auch in einem Wanderbett oder Wirbelbett durchführbar.

Als Katalysatorbeschickung für eine konventionelle heterogen katalysierte Dehydrierung eines zu dehydrierenden Kohlenwasserstoffs (z. B. Propan) mit einem wie beschrieben vergleichsweise geringen Umsatz bei einmaligem Durchgang durch den erfindungsgemäßen Reaktionsraum eignen sich insbesondere die in der DE-A 199 37 107 offenbarten, vor allem beispielhaft offenbarten, Katalysatoren, sowie deren Gemische mit bezüglich der konventionellen heterogen katalysierten Dehydrierung inerten geometrischen Formkörpern.

Nach längerer Betriebsdauer sind vorgenannte Katalysatoren zum Beispiel in einfacher Weise dadurch regenerierbar, dass man bei einer Eintrittstemperatur von 300 bis 600°C (in Extremfällen gegebenenfalls auch bis 750°C), häufig bei 400 bis 550°C, zunächst in ersten Regenerierungsstufen mit Stickstoff und/oder Wasserdampf (bevorzugt) verdünnte Luft durch das Katalysator(fest)bett leitet. Die Katalysatorbelastung mit Regeneriergas kann (bezogen auf die regenerierte Katalysatorgesamtmenge) dabei z. B. 50 bis 10000 h⁻¹ und der Sauerstoffgehalt des Regeneriergases 0,5 bis 20 Vol.-% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (z. B. N₂) zu spülen.

Anschließend ist es in der Regel empfehlenswert, noch mit reinem molekularem Wasserstoff oder mit durch Inertgas (vorzugsweise Wasserdampf und/oder Stickstoff) verdünntem molekularem Wasserstoff (der Wasserstoffgehalt sollte ≥ 1 Vol.-% betragen) im ansonsten gleichen Bedingungsraster zu regenerieren.

Eine konventionelle heterogen katalysierte Kohlenwasserstoff(z. B. Propan)dehydrierung kann beim erfindungsgemäßen Verfahren im erfindungsgemäßen Reaktionsraum bei Katalysatorbelastungen (bezogen auf die eingesetzte Katalysatorgesamtmenge) sowohl mit Ausgangsgas als auch mit darin enthaltenem zu dehydrierendem Kohlenwasserstoff (z. B. Propan) von 100 bis 10000 h⁻¹, häufig 300 bis 5000 h⁻¹, das heißt vielfach 500 bis 3000 h⁻¹ sowohl bei geringem (≤ 30 mol-%) als auch bei hohem (≥ 30 mol-%) Umsatz an zu dehydrierendem Kohlenwasserstoff (z. B. Propan) betrieben werden.

In besonders eleganter Weise lässt sich eine konventionelle erfindungsgemäße heterogen katalysierte Dehydrierung wenigstens eines zu dehydrierenden Kohlenwasserstoffs (z. B. Propan) (sowohl bei Dehydrierumsätzen ≤ 30 mol-% als auch > 30 mol-% (z.B. 40 mol-%, oder 50 mol-%)) in einem erfindungsgemäßen Hordenreaktionsraum verwirklichen.

Ein solcher enthält räumlich aufeinanderfolgend mehr als ein die Dehydrierung katalysierendes Katalysatorbett. Die Katalysatorbettenzahl kann z. B. 1 bis 20, zweckmäßig 2 bis 8, aber auch 3 bis 6 betragen. Vorzugsweise sind die Katalysatorbetten radial oder axial hintereinander angeordnet. Anwendungstechnisch zweckmäßig wird in einem solchen Hordenreaktionsraum der Katalysatorfestbetttyp angewendet.

Im einfachsten Fall sind die Katalysatorfestbetten im Reaktionsraum axial oder in den Ringspalten von zentrisch ineinandergestellten zylindrischen Gitterrosten angeordnet. Es ist jedoch auch möglich, die Ringspalte im Reaktionsraum in Segmenten übereinander anzuordnen und das Gas nach radialem Durchtritt in einem Segment in das nächste darüber- oder darunter liegende Segment zu führen.

In zweckmäßiger Weise wird das Reaktionsgas (Ausgangsgas) auf seinem Weg von einem Katalysatorbett zum nächsten Katalysatorbett zum Beispiel durch Überleiten über mit heißen Gasen erhitzte Wärmetauscherrippen oder durch Leiten durch mit heißen Brenngasen erhitzte Rohre bzw. mit heißen Gasen erhitzte Wärmetauscherplatten, im Hordenreaktionsraum einer Zwischenerhitzung unterworfen.

Wird das erfindungsgemäße Verfahren im Hordenreaktionsraum im übrigen adiabat betrieben, ist es für Dehydrierumsätze (z. B. Propanumsätze) ≤ 30 mol-%, insbesondere bei Verwendung der in der DE-A 199 37 107 beschriebenen Katalysatoren, insbesondere der beispielhaften Ausführungsformen, ausreichend, das Ausgangsgas auf eine Temperatur von 400 bzw. 450 bis 550°C (vorzugsweise 400 bis 500°C) vorerhitzt in den Reaktionsraum zu führen und innerhalb des Hordenreaktionsraums wenigstens in diesem Temperaturbereich zu halten. Das heißt, die gesamte erfindungsgemäße Dehydrierung ist so, wenigstens mit frischen Katalysatoren, bei vergleichsweise gemäßigten Temperaturen zu verwirklichen, was sich für die Standzeit der Katalysatorfestbetten zwischen zwei Regenerierungen als besonders günstig erweist.

Noch geschickter ist es, eine konventionelle heterogen katalysierte Dehydrierung im erfindungsgemäßen Reaktionsraum (wie ebenfalls bereits angesprochen) im wesentlichen autotherm durchzuführen, d.h., die vorstehend geschilderte Zwischenerhitzung auf direktem Weg durchzuführen (autotherme Fahrweise).

Dazu kann dem Reaktionsgas auf seinem Weg durch den erfindungsgemäßen Reaktionsraum z. B. nach Durchströmung des ersten Katalysatorbettes und zwischen den nachfolgenden Katalysatorbetten vorteilhaft in begrenztem Umfang molekularer Sauerstoff zugesetzt werden. Je nach verwendetem Dehydrierkatalysator wird so eine begrenzte Verbrennung der im Reaktionsgas enthaltenen Kohlenwasserstoffe, gegebenenfalls bereits auf der Katalysatoroberfläche abgeschiedener Kohle beziehungsweise kohleähnlicher Verbindungen und/oder von im Verlauf der konventionellen heterogen katalysierten Dehydrierung (z. B. einer Propandehydrierung) gebildetem und/oder dem Reaktionsgas zugesetztem Wasserstoff bewirkt (es kann auch anwendungstechnisch zweckmäßig sein, im Hordenreaktionsraum Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4,788,371, US-A 4,886,928, US-A 5,430,209, US-A 5,530,171, US-A 5,527,979 und US-A 5,563,314 in Betracht; beispielsweise können solche Katalysatorbetten in alternierender Weise zu den Dehydrierkatalysator enthaltenden Betten im Hordenreaktionsraum untergebracht sein)). Die dabei freigesetzte Reaktionswärme ermöglicht so (quasi adiabate Reaktorgestaltung) auf quasi autotherme Weise eine nahezu isotherme (interne Temperaturlenkung) Betriebsweise der heterogen katalysierten (z. B. Propan)Dehydrierung. Mit zunehmender gewählter Verweilzeit des Reaktionsgases im Katalysatorbett ist so eine (z. B. Propan)Dehydrierung bei abnehmender oder im wesentlichen konstanter Temperatur möglich, was besonders lange Standzeiten zwischen zwei Regenerierungen ermöglicht.

Durch eine wie beschrieben durchgeführte Folgeverbrennung von im Verlauf der Dehydrierung gebildetem molekularem Wasserstoff wird aus einer nichtoxidativen konventionellen heterogen katalysierten Dehydrierung eine im Sinne der vorliegenden Anmeldung oxidative konventionelle heterogen katalysierte Dehydrierung.

In der Regel sollte eine wie vorstehend beschriebene Sauerstoffeinspeisung so vorgenommen werden, dass der Sauerstoffgehalt des Reaktionsgases, bezogen auf die darin enthaltene Menge an zu dehydrierendem Kohlenwasserstoff und dehydriertem Kohlenwasserstoff (z. B. Propan und Propylen), 0,01 bzw. 0,5 bis 30 Vol.-% beträgt. Als Sauerstoffquelle kommen dabei sowohl reiner molekularer Sauerstoff oder mit Inertgas, zum Beispiel CO, CO₂, N₂, Edelgase, verdünnter Sauerstoff, insbesondere aber auch Luft, in Betracht. Die resultierenden Verbrennungsgase wirken in der Regel zusätzlich verdünnend und fördern dadurch die heterogen katalysierte (z. B. Propan) Dehydrierung.

Die Isothermie einer konventionellen heterogen katalysierten (z. B. Propan)Dehydrierung lässt sich dadurch weiter verbessern, dass man im Hordenreaktionsraum in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Ausgangsgas bzw. den Ausgangsgasstrom für eine konventionelle heterogen katalysierte (z. B. Propan)Dehydrierung im erfindungsgemäßen Reaktionsraum auf die benötigte Reaktionstemperatur zu erwärmen, besteht auch darin, einen Teil des darin enthaltenen zu dehydrierenden Kohlenwasserstoffs (z. B. des Propans) und/oder H₂ mittels im Ausgangsgas enthaltenem molekularem Sauerstoff beim Eintritt in den Reaktionsraum zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die (für die Dehydrierung) gewünschte Reaktionstemperatur zu bewirken. Die resultierenden Verbrennungsprodukte, wie CO₂, H₂O sowie das den für die Verbrennung benötigten molekularen Sauerstoff gegebenenfalls begleitende N₂ bilden vorteilhafte inerte Verdünnungsgase.

Besonders elegant lässt sich die vorgenannte Wasserstoffverbrennung wie in der WO 03/076370 bzw. DE-A 102 11 275 beschrieben realisieren. D.h., in einem Verfahren der kontinuierlichen konventionellen oxidativen heterogen katalysierten partiellen Dehydrierung von zu dehydrierendem Kohlenwasserstoff (z. B. im Propan) im erfindungsgemäßen Reaktionsraum, bei dem man
- dem Reaktionsraum wenigstens einen, wenigstens einen zu dehydrierenden Kohlenwasserstoff (z. B. Propan), molekularen Sauerstoff, molekularen Wasserstoff und gegebenenfalls Wasserdampf enthaltenden Ausgangsgasstrom kontinuierlich zuführt,
- im Reaktionsraum den wenigstens einen zu dehydrierenden Kohlenwasserstoff durch wenigstens ein im Reaktionsraum befindliches Katalysatorbett führt, an welchem durch konventionelle heterogen katalysierte Dehydrierung molekularer Wasserstoff und wenigstens partiell wenigstens ein dehydrierter Kohlenwasserstoff (z. B. Propylen) gebildet werden,
- dem Reaktionsgas nach seinem Eintritt in den erfindungsgemäßen Reaktionsraum auf seinem Weg durch den Reaktionsraum gegebenenfalls weiteres molekularen Sauerstoff enthaltendes Gas zusetzt,
- der molekulare Sauerstoff im Reaktionsraum im Reaktionsgas enthaltenen molekularen Wasserstoff wenigstens teilweise zu Wasserdampf oxidiert, und
- dem Reaktionsraum wenigstens einen Produktgasstrom kontinuierlich entnimmt, der molekularen Wasserstoff, Wasserdampf, dehydrierten Kohlenwasserstoff (z. B. Propylen) und zu dehydrierenden Kohlenwasserstoff (z. B. Propan) enthält,
das dadurch gekennzeichnet ist, dass der dem erfindungsgemäßen Reaktionsraum entnommene wenigstens eine Produktgasstrom in zwei Teilmengen identischer Zusammensetzung aufgeteilt und eine der beiden Teilmengen als Dehydrierkreisgas in den dem erfindungsgemäßen Reaktionsraum zugeführten wenigstens einen Ausgangsgasstrom rückgeführt und die andere Teilmenge anderweitig weiterverwendet (z. B. zum Zweck einer heterogen katalysierten partiellen Oxidation von im Reaktionsraum gebildetem dehydriertem Kohlenwasserstoff) wird.

Beispielsweise kann das Produktgas einer erfindungsgemäßen, oxidativen oder nicht oxidativen heterogen katalysierten Dehydrierung von Propan zu Propylen die folgenden Gehalte aufweisen

| | |
|---|---|
| Propan | 25 bis 60 Vol.-%, |
| Propylen | 8 bis 25 Vol.-%, |
| H₂ | ≥ 0 bis 25 Vol.-% und |
| CO₂ | ≥ 0 bis 30 Vol.-%. |

In den vorstehenden Ausführungen wurde in individualisierter Form als nichtoxidativ oder oxidativ in konventioneller Weise heterogen katalysiert zu dehydrierender Kohlenwasserstoff stets Propan benannt. Selbstredend sind die beschriebenen Verfahrensweisen aber auch auf alle anderen eingangs dieser Schrift als zu dehydrierende Kohlenwasserstoffe aufgeführten Verbindungen anwendbar. Im besonderen seien unter diesen nochmals Butan (zu Buten und/oder Butadien; insbesondere iso-Butan zu iso-Buten) sowie von Butenen zu Butadien genannt.

Ganz allgemein enthält der wenigstens eine Ausgangsgasstrom einer erfindungsgemäßen, oxidativen oder nicht oxidativen heterogen katalysierten Dehydrierung in der Regel ≥ 5 Vol.-% des zu dehydrierenden Kohlenwasserstoffs (z.B. Propan). Daneben kann er z.B. enthalten:
a) N₂ und H₂O;
b) N₂, O₂ und H₂O;
c) N₂, O₂, H₂O und H₂;
d) N₂, O₂, H₂O, H₂ und CO₂.

Wie bereits erwähnt, wird sich an das erfindungsgemäße Verfahren vielfach ein Verfahren der heterogen katalysierten Partialoxidation von erzeugtem dehydriertem Kohlenwasserstoff (z. B. Propylen zu Acrolein und/oder Acrylsäure), vorzugsweise in Begleitung vom nicht umgesetzten zu dehydrierenden Kohlenwasserstoff (z. B. Propan) als Inertgas anschließen. Dabei wird man den aus dem erfindungsgemäßen Reaktionsraum (kontinuierlich) entnommenen Produktgasstrom als solchen oder nach Abtrennung wenigstens einer Teilmenge seiner vom dehydrierten Kohlenwasserstoff (z. B. Propylen) und vom (nicht umgesetzten) zu dehydrierenden Kohlenwasserstoff (z. B. Propan) verschiedenen Bestandteile (z. B. H₂, H₂O, N₂, etc.) zur Beschickung wenigstens eines Oxidationsreaktors verwenden, und den im Beschickungsgasgemisch enthaltenen dehydrierten Kohlenwasserstoff (z. B. Propylen) einer selektiven heterogen katalysierten partiellen Gasphasenoxidation mit molekularem Sauerstoff zu einem das (Partialoxidationsprodukt) Zielprodukt (z. B. Acrolein, oder Acrylsäure oder deren Gemisch), sowie in der Regel nicht umgesetzten zu dehydrierenden Kohlenwasserstoff (z. B. Propan), überschüssigen molekularen Sauerstoff und gegebenenfalls nicht umgesetzten dehydrierten Kohlenwasserstoff (z. B. Propylen) enthaltenden Produktgasgemisch B unterwerfen.

In einer nachfolgenden Trennzone B wird man im Produktgasgemisch B enthaltenes Zielprodukt (z. B. Acrolein, oder Acrylsäure, oder deren Gemisch) abtrennen, und von dem dabei verbleibenden, nicht umgesetzten zu dehydrierenden Kohlenwasserstoff (z. B. Propan), molekularen Sauerstoff sowie gegebenenfalls nicht umgesetzten dehydrierten Kohlenwasserstoff (z. B. Propylen) enthaltenden Restgas wenigstens eine nicht umgesetzten zu dehydrierenden Kohlenwasserstoff (z. B. Propan) und gegebenenfalls nicht umgesetzten molekularen Sauerstoff sowie gegebenenfalls nicht umgesetzten dehydrierten Kohlenwasserstoff (z. B. Propylen) enthaltende Teilmenge als Partialoxidationskreisgas in das erfindungsgemäße Verfahren rückführen (z. B. als Bestandteil des Ausgangsgasstroms).

Handelt es sich beim erfindungsgemäßen Verfahren (aber auch sonst) z. B. um eine oxidative konventionelle heterogen katalysierte partielle Dehydrierung von Propan zu Propylen, und bei der sich anschließenden Partialoxidation um diejenige von Propylen zu Acrolein, oder zu Acrylsäure, oder zu deren Gemisch, kann der dem erfindungsgemäßen Reaktionsraum zugeführte wenigstens eine Ausgangsgasstrom z. B. als wesentliche Gehalte enthalten:

| | |
|---|---|
| Propylen | ≥ 0 bis 20 bzw. bis 10, häufig 0 bis 6 Vol.-%, |
| Acrolein | ≥ 0 bis 1, vielfach 0 bis 0,5, häufig 0 bis 0,25 Vol.-%, |
| Acrylsäure | ≥ 0 bis 0,25, vielfach 0 bis 0,05, häufig 0 bis 0,03 Vol.-%, |
| COₓ | ≥ 0 bis 20 bzw. bis 5, vielfach 0 bis 3, häufig 0 bis 2 Vol.-%, |
| Propan | 5 bis 50, vorzugsweise 10 bis 20 Vol.-%, |
| Stickstoff | 20 bzw. 30 bis 80, vorzugsweise 50 bis 70 Vol.-%, |
| Sauerstoff | ≥ 0 bis 5, vorzugsweise 1,0 bis 2,0 Vol.-%, |
| H₂O | ≥ 0 bis 20, vorzugsweise 5,0 bis 10,0 Vol.-%, und |
| H₂ | ≥ 0, häufig ≥ 0,01, oft ≥ 0,05 bis 10, vorzugsweise 1 bis 5 Vol.-%. |

In geringen Menge (etwa vergleichbar den möglichen Acrylsäuregehalten) kann auch Essigsäure enthalten sein.

Üblicherweise erfolgt die Abtrennung von Zielprodukt (z. B. Acrylsäure) aus dem Produktgasgemisch B dadurch, dass man das Zielprodukt (z. B. die Acrylsäure) in die kondensierte Phase überführt. Dies kann durch absorptive und/oder kondensative (kühlen) Maßnahmen erfolgen. Als Absorptionsmittel kommen im Fall von Acrylsäure als Zielprodukt z. B. Wasser, wässrige Lösungen oder hochsiedende (T_{siede} > T_{Siede} von Acrylsäure bei 1 atm), insbesondere hydrophobe, organische Lösungsmittel in Betracht. Besonders bevorzugt erfolgt das Überführen in die kondensierte Phase im Fall der Acrylsäure durch fraktionierende Kondensation das Produktgasgemisches B. Vorzugsweise erfolgt die absorptive und/oder kondensative Überführung der Acrylsäure aus dem Produktgasgemisch B in die kondensative Phase in trennwirksame Einbauten enthaltenden Kolonnen, in denen das Produktgasgemisch normalerweise von unten nach oben aufsteigend geführt wird. Das Absorptionsmittel wird in der Regel am Kolonnenkopf aufgegeben, an dem das Restgas normalerweise aus der Kolonne entlassen wird.

Die weitere Abtrennung der Acrylsäure aus der kondensierten Phase erfolgt in der Regel in der gewünschten Reinheit unter Anwendung wenigstens eines thermischen Trennverfahrens. Darunter werden solche Verfahren verstanden, bei denen wenigstens zwei voneinander verschiedene stoffliche Phasen (z. B. flüssig/flüssig; gasförmig/flüssig; fest/flüssig; gasförmig/fest etc.) erzeugt und miteinander in Kontakt gebracht werden. Aufgrund des zwischen den Phasen bestehenden Ungleichgewichts findet zwischen denselben ein Wärme- und Stoffaustausch statt, der letztlich die gewünschte Auftrennung (Abtrennung) bedingt. Die Bezeichnung "thermische Trennverfahren" reflektiert dabei, dass es entweder des Entzuges oder der Zufuhr von Wärme bedarf, um die Ausbildung der stofflichen Phasen zu erzeugen und/oder dass der Entzug oder die Zufuhr von thermischer Energie den Stoffaustausch begünstigt bzw. aufrechterhält.

Erfindungsgemäß bevorzugt umfasst das wenigstens eine thermische Trennverfahren wenigstens eine kristallisative Abtrennung aus flüssiger Phase. Erfindungsgemäß zweckmäßig ist die wenigstens eine kristallisative Abtrennung der Acrylsäure eine Suspensionskristallisation und mit Vorteil wird das Suspensionskristallisat mit geschmolzenem, zuvor abgetrenntem und gewaschenem Kristallisat in einer Waschkolonne (eine gravimetrische, oder eine mechanische, oder eine hydraulische Waschkolonne; letztere ist erfindungsgemäß bevorzugt) gewaschen. Im übrigen kommen als thermische Trennverfahren z. B. extraktive, desorptive, kristallisative, rektifikative, azeotrop-destillative, azeotrop-rektifikative, destillative und/oder Strippverfahren in Betracht. In der Regel wird man zur Gewinnung von reiner Acrylsäure Kombinationen von verschiedenen der genannten thermischen Trennverfahren anwenden.

An die beschriebene Abtrennung der Acrylsäure kann sich ein Verfahren der radikalischen Polymerisation (insbesondere zur Herstellung von Wasser superabsorbierenden Polyacrylsäuren und/oder deren teil- bzw. vollneutralisierten Alkali(vorzugsweise Na)metallsalzen) anschließen, in welchem abgetrennte Acrylsäure zur Herstellung von Polymerisaten radikalisch einpolymerisiert wird.

Auch kann sich an die beschriebene Abtrennung der Acrylsäure ein Verfahren zur Herstellung von Acrylsäureestern anschließen, in welchem abgetrennte Acrylsäure mit Alkoholen (vorzugsweise Alkanolen, besonders bevorzugt C₁- bis C₁₂-Alkanolen) (in der Regel säurekatalysiert) verestert wird.

An das Verfahren der Veresterung kann sich wiederum ein Verfahren der radikalischen Polymerisation anschließen, in welchem so hergestellter Acrylsäureester einpolymerisiert wird.

Sieht man von der erfindungsgemäßen Besonderheit des erfindungsgemäßen Reaktionsraumes ab, sind erfindungsgemäße Verfahren zur Herstellung von Propylen aus Propan als Propylenquelle für Partialoxidationen desselben zur Herstellung von Acrolein und/oder Acrylsäure vorbekannt, einschließlich einer Kreisgasführung von Oxidationskreisgas und gegebenenfalls Dehydrierkreisgas. Beispielsweise findet man solche mehrstufigen Verfahren beschrieben in den Schriften DE-A 10 2005 022 798, DE-A 102 46 119, DE-A 102 45 585, DE-A 10 2005 049 699, DE-A 10 2004 032 129, DE-A 10 2005 013 039, DE-A 10 2005 010 111, DE-A 10 2005 009 891, DE-A 102 11 275, EP-A 117 146, US 3,161,670, DE-A 33 13 573, WO 01/96270, DE-A 103 16 039, DE-A 10 2005 009 885, DE-A 10 2005 052 923, DE-A 10 2005 057 197, WO 03/076370, DE-A 102 45 585, DE-A 22 13 573, US 3,161,670 und dem in diesen Schriften zitierten Stand der Technik. Die DE-A 102 19 686 offenbart die entsprechende Vorgehensweise im Fall einer Herstellung von Methacrolein und/oder Methacrylsäure.

Absorptive und/oder kondensative Verfahren zur Überführung von Acrylsäure aus einem Produktgasgemisch B in die kondensierte Phase finden sich ebenfalls ausführlich im Stand der Technik beschrieben. Angeführt seien die Schriften DE-A 103 36 386, DE-A 196 31 645, DE-A 195 01 325, EP-A 982 289, DE-A 198 38 845, WO 02/076917, EP-A 695 736, EP-A 778 225, EP-A 1 041 062, EP-A 982 287, EP-A 982 288, US-A 2004/0242826, EP-A 792 867, EP-A 784 046, EP-A 695 736 (insbesondere absorptive) sowie WO 04/035514, DE-A 199 24 532, DE-A 198 14 387, DE-A 197 40 253, DE-A 197 40 252 und DE-A 196 27 847 (insbesondere kondensativ).

Ferner finden sich solche absorptiven und/oder kondensativen Abtrennungen von Acrylsäure aus Produktgasgemischen B auch beschrieben in den Schriften EP-A 1 338 533, EP-A 1 388 532, DE-A 102 35 847, WO 98/01415, EP-A 1 015 411, EP-A 1 015 410, WO 99/50219, WO 00/53560, WO 02/09839, DE-A 102 35 847, WO 03/041833, DE-A 102 23 058, DE-A 102 43 625, DE-A 103 36 386, EP-A 854 129, US 4,317,926, DE-A 198 37 520, DE-A 196 06 877, DE-A 195 01 325, DE-A 102 47 240, DE-A 197 40 253, EP-A 695 736, EP-A 1 041 062, EP-A 117 146, DE-A 43 08 087, DE-A 43 35 172, DE-A 44 36 243, DE-A 103 32 758 sowie DE-A 199 24 533. Grundsätzlich kann dabei aber auch wie in der DE-A 103 36 386, DE-A 101 15 277, DE-A 196 06 877, EP-A 920 408, EP-A 1 068 174, EP-A 1 066 239, EP-A 1 066 240, WO 00/53560, WO 00/53561, DE-A 100 53 086, WO 01/96271, DE-A 10 2004 032 129, WO 04/063138, WO 04/35514, DE-A 102 43 625 und DE-A 102 35 847 beschrieben vorgegangen werden.

Handelt es sich beim zu dehydrierenden Kohlenwasserstoff für das erfindungsgemäße Verfahren um Propan, wird man selbigen vorzugsweise als Roh-Propan gemäß der Lehre der DE-A 102 45 585 dem wenigstens einen Ausgangsgasstrom zuführen.

Generell wird der wenigstens eine Ausgangsgasstrom wenigstens zu 5 Vol.-% an zu dehydrierendem Kohlenwasserstoff enthalten. Häufig liegt dieser Volumenanteil bei entsprechend bezogenen Werten von ≥ 10 Vol.-%, oft ≥ 15 Vol.-% und meist ≥ 20 Vol.-% bzw. ≥ 25 Vol.-%, oder ≥ 30 Vol.-%. In der Regel liegt dieser Volumenanteil jedoch bei in gleicher Weise bezogenen Werten von ≤ 90 Vol.-%, meist ≤ 80 Vol.-% und oft ≤ 70 Vol.-%. Vorstehende Angaben gelten insbesondere im Fall von Propan als zu dehydrierendem Kohlenwasserstoff und Propylen als dehydriertem Kohlenwasserstoff. Selbstredend treffen sie aber auch dann zu, wenn iso-Butan der zu dehydrierende Kohlenwasserstoff und iso-Buten der dehydrierte Kohlenwasserstoff ist.

In bemerkenswerter Weise ist zur Durchführung einer erfindungsgemäßen konventionellen (oxidativen oder nichtoxidativen) heterogen katalysierten Dehydrierung, insbesondere für eine adiabate Betriebsweise, der Innenraum eines einfachen Schachtofens ("Schachtofenreaktor") als das wenigstens eine Katalysatorbett (z.B. das wenigstens eine Katalysatorfestbett) enthaltender erfindungsgemäßer Reaktionsraum ausreichend, der vom Ausgangsgasstrom axial und/oder radial durchströmt wird.

Im einfachsten Fall handelt es sich dabei zum Beispiel um einen im wesentlichen zylindrischen Behälter, dessen Innendurchmesser 0,1 bis 10 m, eventuell 0,5 bis 5 m beträgt und in welchem das wenigstens eine Katalysatorfestbett auf einer Trägervorrichtung (zum Beispiel ein Gitterrost) aufgebracht ist. Der mit Katalysator beschickte Reaktionsraum, dessen erfindungsgemäße Einhüllende im adiabaten Betrieb wärmeisoliert ist, wird dabei zweckmäßig mit dem heißen, den zu dehydrierenden Kohlenwasserstoff (z.B. das Propan) enthaltenden Ausgangsgasstrom axial durchströmt. Die Katalysatorgeometrie kann dabei sowohl kugelförmig als auch ringförmig oder strangförmig sein. Da im ebenda beschriebenen Fall der Reaktionsraum durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des den zu dehydrierenden Kohlenwasserstoff (z.B. das Propan) enthaltenden Reaktionsgases kann der erfindungsgemäße Reaktionsraum zum Beispiel zwei konzentrisch ineinander gestellte zylindrische Gitterroste enthalten und die Katalysatorschüttung in deren Ringspalt angeordnet sein. Im adiabaten Fall wäre die ihn umgebende Einhüllende (die Mantelhülle) gegebenenfalls wiederum thermisch isoliert. Im Fall eines axial durchströmten im wesentlichen zylindrischen Schachtofens ist es für das erfindungsgemäße Verfahren im Fall einer adiabaten Betriebsweise vorteilhaft, wenn die Ausdehnung A des Reaktionsraums senkrecht zur zylindrischen Achse wenigstens das 5-fache, vorzugsweise wenigstens das 10-fache und besonders bevorzugt wenigstens das 15-fache der Schütthöhe S des wenigstens einen Katalysatorbettes in axialer Richtung beträgt. In der Regel wird das vorgenannte Verhältnis von A:S jedoch ≤ 200, üblicherweise ≤ 150 und meist ≤ 100 betragen.

Vor dem Hintergrund des bisher Gesagten umfasst vorliegende Erfindung als einen erfindungsgemäßen Gegenstand insbesondere eine einen Innenraum I (den Reaktionsraum) umschließende (materielle) Einhüllende E, mit wenigstens einer ersten Öffnung O1 zur Zufuhr wenigstens eines Gasstroms (Stoffstroms) S in den Innenraum I und wenigstens einer zweiten Öffnung 02 zur Abfuhr A (Entnahme) eines dem Innenraum zuvor über die wenigstens eine erste Öffnung O1 zugeführten Gasstroms (Stoffstroms) S aus dem Innenraum I, wobei die Oberfläche der Einhüllenden E auf ihrer den Innenraum I berührenden Seite wenigstens teilweise in einer Schichtdicke d von wenigstens 1 mm aus einem Stahl S gefertigt ist, der die nachfolgende Elementzusammensetzung aufweist:
18 bis 30 Gew.-% Cr,
9 bis 36 Gew.-% Ni,
1 bis 3 Gew.-% Si,
0,1 bis 0,3 Gew.-% N,
≥ 0 bis 0,15 Gew.-% C,
≥ 0 bis 4 Gew.-% Mn,
≥ 0 bis 4 Gew.-% Al,
≥ 0 bis 0,05 Gew.-% P,
≥ 0 bis 0,05 Gew.-% S, und
≥ 0 bis 0,1 Gew.-% eines oder mehrere seltener Erdmetalle, und im übrigen Fe und herstellungsbedingte Verunreinigungen, wobei die Prozentangaben jeweils auf das Gesamtgewicht bezogen sind.

Vorteilhaft beträgt die Gesamtmenge der herstellungsbedingten Verunreinigungen ganz generell und in gleicher Weise bezogen ≤ 1 Gew.-%, vorzugsweise ≤ 0,75 Gew.-%, besonders bevorzugt ≤ 0,5 Gew.-% und ganz besonders bevorzugt ≤ 0,25 Gew.-%. In der Regel wird die Gesamtmenge der herstellungsbedingten Verunreinigungen des Stahls jedoch ≥ 0,1 Gew.-% betragen. Erfindungsgemäß bevorzugtes seltenes Erdmetall ist Ce. Erfindungsgemäß bevorzugt weist der relevante Stahl daher ≥ 0 bis 0,1 Gew.-% Ce oder Ce und eines oder mehrere von Ce verschiedene seltene Erdmetalle auf. Der Innenraum I aller Einhüllenden E eignet sich nach der Aufnahme wenigstens eines Dehydrierkatalysator enthaltenden Katalysatorbetts zur Durchführung von erfindungsgemäßen Verfahren (insbesondere solchen der adiabaten heterogen katalysierten konventionellen nicht oxidativen oder oxidativen Dehydrierung von Propan zu Propylen).

Erfindungsgemäß vorteilhaft ist die Oberfläche der Einhüllenden E auf ihrer den Innenraum I berührenden Seite zu wenigstens 10 %, vorzugsweise zu wenigstens 20 %, oder zu wenigstens 30 %, ganz besonders bevorzugt zu wenigstens 40 %, oder zu wenigstens 50 %, noch vorteilhafter zu wenigstens 60 % oder zu wenigstens 70 %, besser zu wenigstens 80 % oder zu wenigstens 90 % und am Besten zu wenigstens 95 % oder zu wenigstens 100 % ihrer Gesamtfläche in einer Schichtdicke d von wenigstens 1 mm aus dem relevanten Stahl S gefertigt.

Erfindungsgemäß vorteilhaft beträgt vorgenanntes d wenigstens 2 mm, oder wenigstens 3 mm, oder wenigstens 4 mm, oder wenigstens 5 mm. Selbstverständlich kann d aber auch ≥ 5 mm bis 30 mm, oder bis zu 25 mm, oder bis zu 20 mm, oder bis zu 15 mm, oder bis zu 10 mm betragen. Ganz besonders bevorzugt ist die Einhüllende E in ihrer Gesamtheit bzw. zu wenigstens 80 % ihres Gewichtes, besser zu wenigstens 90 % oder zu wenigstens 95 % ihrer Gewichtes aus einem erfindungsgemäßen Stahl S gefertigt.

Erfindungsgemäß vorteilhaft beträgt das Volumen des Innenraums I (leer gerechnet) 5 m³ bis 500 m³, oft 10 m³ bis 400 m³, häufig 20 m³ bis 300 m³, vielfach 30 m³ bis 200 m³, oder 50 m³ bis 150 m³.

Selbstredend kommen für den Stahl S der Einhüllenden E auch alle anderen in dieser Schrift bereits genannten erfindungsgemäßen Stähle S in Betracht und dies in der in dieser Schrift bereits im Vorstehenden gegebenen Bevorzugungsabstufung. Mit Vorteil ist der Stahl S auf seiner den Innenraum I berührenden Seite alonisiert, alitiert und/oder aluminiert.

Im besonderen betrifft vorliegende Erfindung Einhüllende E, deren Innenraum I wenigstens einen für eine heterogen katalysierte partielle Dehydrierung eines zu dehydrierenden Kohlenwasserstoffs geeigneten Katalysator (Dehydrierkatalysator) enthält. Als solche Katalysatoren kommen insbesondere alle in dieser Schrift aufgeführten Dehydrierkatalysatoren in Betracht.

Die im Innenraum der Einhüllenden E enthaltene Katalysatormenge kann 100 kg bis 500 t (Tonnen (metrische)) oder 200 kg bis 400 t, oder 300 kg bis 300 t, oder 400 kg bis 200 t, bzw. 500 kg bis 150 t, oder 1 t bis 100 t, oder 10 t bis 80 t, oder 20 t bis 60 t betragen. Nicht eingerechnet sind hierbei die Katalysatoren gegebenenfalls verdünnende ausschließlich inerte Formkörper.

Ferner betrifft vorliegende Erfindung Einhüllende E, deren Innenraum I wenigstens einen (Gitter)Rost enthält (vorzugsweise wenigstens zwei zentrisch ineinander gestellte (Gitter)Roste). Mit Vorteil weist (enthält) die Einhüllende E ein ringförmiges (hohlkreiszylinderförmiges) Segment R (bzw. einen solchen Abschnitt) auf (wobei der Ringinnenraum I_{R} einen Teil des Innenraumes I bildet).

Ist D die Hälfte der Differenz zwischen dem Außendurchmesser A (ohne äußere Wärmedämmung gerechnet) und dem Innendurchmesser des ringförmigen Segments R, so beträgt das Verhältnis V₁ von D zu A erfindungsgemäß bevorzugt 1 : 10 bis 1 : 1000, vielfach 1 : 20 bis 1 : 800, oft 1 : 30 bis 1 : 600, vielfach 1 : 40 bis 1 : 500, oder 1 : 50 bis 1 : 400, bzw. 1 : 60 bis 1 : 300, in vielen Fällen aber auch 1 : 70 bis 1 : 200, oder 1 : 80 bis 1 : 150.

Das Verhältnis V₂ aus der Höhe H (der Abstand der beiden das ringförmige Segment R begrenzenden parallelen kreisförmigen Ebenen) und A (V₂=H : A) kann sowohl > 1, oder = 1, oder < 1 betragen.

Beträgt V₂>1, beträgt es in typischer Weise 2 bis 10, häufig 4 bis 8 und oft 6. Beträgt V₂<1, beträgt es in typischer Weise 0,01 bis 0,95, häufig 0,02 bis 0,9, oft 0,03 bis 0,8, vielfach 0,05 bis 0,7, oder 0,07 bis 0,5 bzw. 0,1 bis 0,4. Mögliche Werte für V₂ sind somit auch 0,2 und 0,3.
Ist V₂>1, wird A meist ≥ 0,5 m bis 5 m, häufig 1 m bis 4 m, und zweckmäßig 2 m bis 3 m betragen.
Ist V₂<1, wird A typisch ≥ 0,5 m bis 8 m, vorzugsweise 2 m bis 6 bzw. 7 m und oft 2,5 m bis 5 m betragen.
Ist V₂=1 betragen typische Außendurchmesser A 0,5 bis 8 m, oder 2 bis 6 bzw. 7 m, bzw. 2,5 m bis 5 m.

Ist V₂>1, eignet sich der ringförmige Innenraum (der Ringinnenraum) des ringförmigen Segments R der Einhüllenden E insbesondere zur Gestaltung eines für das erfindungsgemäße Verfahren besonders geeigneten radial durchströmten erfindungsgemäßen Hordenreaktionsraums. Dazu enthält der ringförmige Innenraum I_{R} in zweckmäßiger Weise zwischen den Ringspalten von zentrisch ineinandergestellten Gitterrosten Katalysatorfestbetten aufgeschüttet, wobei die Ringspalte mit Vorteil abschnittsweise so übereinander angeordnet sind, dass ein sie durchströmendes Reaktionsgas nach radialem Durchtritt in einem Abschnitt in den nächsten darüber - oder darunter liegenden Abschnitt geführt wird. Die Anzahl vorgenannter Katalysatorbetten kann 1 bis 20, zweckmäßig 2 bis 8 und bevorzugt 3 bis 6 betragen. D.h., erfindungsgemäß bevorzugt sind Einhüllende E mit einem Ringinnenraum I_{R}, die abschnittsweise übereinander angeordnete Ringspalte aus jeweils zentrisch ineinandergestellten Gitterrosten aufweisen und deren V₂>1 beträgt.

Ist V₂<1, eignet sich der ringförmige Innenraum (der Ringinnenraum) des ringförmigen Segments R der Einhüllenden E insbesondere zur Gestaltung eines für das erfindungsgemäße Verfahren besonders geeigneten axial durchströmten erfindungsgemäßen Hordenreaktionsraums. Dazu enthält der ringförmige Innenraum I_{R} in zweckmäßiger Weise auf axial (d.h., längs der Ringachse, bzw. Zylinderachse) hintereinander angeordneten Gitterrosten Katalysatorbetten aufgeschüttet, die vom Reaktionsgas nacheinander durchströmt werden können. Die Anzahl vorgenannter Katalysatorbetten kann 1 bis 20, zweckmäßig 2 bis 8 und bevorzugt 3 bis 6 betragen. In der Regel sind sie äquidistant angeordnet.

D.h., erfindungsgemäß bevorzugt sind Einhüllende E mit einem Ringinnenraum I_{R}, die axial hintereinander angeordnete Gitterroste enthalten und deren V₂ < 1 beträgt. Letzteres gilt insbesondere dann, wenn H 2 m bis 4 m (vorzugsweise 3 bis 4 m, bzw. 3,50 m) und der Innendurchmesser des ringförmigen Segments R (bei einer Wanddicke von 1 bis 4 cm) 5,90 m bis 6,30 m beträgt. Zweckmäßig beträgt die Anzahl der axial hintereinander angeordneten Katalysatorbetthorden im vorgenannten Fall drei. Die Schütthöhe eines Katalysatorbetts (z.B. des Katalysators gemäß Bsp. 4 der DE-A 10219879) wird zweckmäßig 10 bis 60 cm (z.B. 30 cm) betragen.

Anwendungstechnisch zweckmäßig werden die beiden das ringförmige Segment R begrenzenden parallelen kreisförmigen Ebenen durch je eine Haube abgeschlossen (zur Einhüllenden E ergänzt). Prinzipiell können die Hauben die Gestalt eines flachen Bodens (Deckels) aufweisen oder auch gewölbt sein. Erfindungsgemäß bevorzugt sind auf beiden Seiten des ringförmigen Segments R gewölbte Hauben. Dabei kann die Wölbung Korbbogenform gemäß DIN 28013 oder Klöpperform gemäß DIN 28011 aufweisen. Die Wölbung der unteren Haube wird normalerweise vom Innenraum I_{R} wegweisen (nach außen gewölbt, konvex, sein). Die Wölbung der oberen Haube kann relativ zum Innenraum I_{R} sowohl konvex oder konkav sein. In anwendungstechnisch einfacher Weise sind beide Hauben relativ zum Innenraum I_{R} konvex gewölbt. Die wenigstens eine erste Öffnung O1 befindet sich in diesem Fall zweckmäßig im Zentrum der oberen Haube und die wenigstens eine zweite Öffnung 02 befindet sich in diesem Fall zweckmäßig im Zentrum der unteren Haube. Vorzugsweise sind die Öffnungen O1 und O2 kreisförmig. Ihre Querschnitte werden anwendungstechnisch zweckmäßig so gewählt, dass ihr Verhältnis dem Verhältnis der über diese ins Auge gefassten zu- bzw. abströmenden Volumenströme entspricht. Bei einer Höhe H von 2 m bis 4 m (vorzugsweise 3 bis 4 m, bzw. 3,50 m) und einem Innendurchmesser des ringförmigen Segments von 5,90 bis 6,30 m kann der Durchmesser der wenigstens einem ersten Öffnung O1 z.B. typisch 1200 mm und der Durchmesser der wenigstens einen zweiten Öffnung 02 z.B. typisch 1400 mm betragen. Anwendungstechnisch vorteilhaft ist die Wanddicke der unteren Haube stärker als D des ringförmigen Segments R. In diesem Fall kann der über D hinausragende Teil der Haubenwand die im ringförmigen Segment R z.B. axial hintereinander angeordneten (aufeinander gestapelten) Gitterroste (bzw. Katalysatorbetten) tragen.

Die einzelnen ringförmigen Gitterroste können aber auch aus einheitlichen (voneinander getrennten) Kreissektoren (Gitterrostsektoren) zusammengesetzt (zusammengefügt) sein (wie der Querschnitt einer Organgenscheibe). Anwendungstechnisch bevorzugt sind Zwölftel-, oder Achtel-, oder Sechstel-, oder Viertel-, oder Drittelkreissektoren. Die Gitterrostkreissektoren können dabei ihrerseits auf offenen Gerüstkreissektoren aufliegen. Die untersten Gerüstkreissektoren können der Wölbung der unteren Haube angepasst fortgeführt und in die konvexe Wölbung der unteren Haube als die Gerüstkreissektoren tragendes Element aufgelegt werden. Die Gerüstkreissektoren von darüber befindlichen Gitterrostsektoren können dann jeweils auf diejenigen der unmittelbar darunter befindlichen Katalysatorhorde aufgelegt werden.

Prinzipiell können Einhüllende E aus dem ringförmigen Segment R und den beiden dieses abschließenden Hauben nahtlos gefertigt sein. In der Regel werden das Segment R und die Hauben jedoch separat gefertigt und müssen nachfolgend weitestgehend gasdicht (Gasleckage <10⁻⁴ mbar •l/s) miteinander verbunden werden. Im Fall der unteren Haube wird diese Verbindung mit dem ringförmigen Segment R vorzugsweise durch Anschweißen erfolgen. Im Fall der oberen Haube wird das Verbinden mit dem ringförmigen Segment R jedoch mit Vorteil durch Anflanschen vorgenommen (die Abnehmbarkeit der oberen Haube erleichtert die Befüllung mit bzw. die Entnahme von Katalysator, z.B. im Fall eines Teilkatalysatorwechsels oder im Fall eines Vollkatalysatorwechsels). Besonders bevorzugt ist eine Flanschverbindung mit Schweißlippendichtung (letztere wird erfindungsgemäß bevorzugt aus Stahlblechen eines Stahls S gefertigt, die eine Dicke von ca. 2 mm aufweisen).

Erfindungsgemäß bevorzugt werden die Katalysatorformkörper nicht unmittelbar auf die Gitterroste aufgebracht. Dies rührt daher, dass die Katalysatorformkörper üblicherweise eine geringere Ausdehnung als die Maschenweite des Gitterrosts aufweisen. In zweckmäßiger Weise wird daher auf einen Gitterrost zunächst eine Schicht (Höhe: 1 bis 10 cm, vorzugsweise 5 bis 10 cm) aus Steatitkugeln des Durchmessers 3 bis 10 cm, häufig 4 bzw. 5 bis 8 cm aufgegeben (bevorzugt wird Steatit C220 der Fa. CeramTec verwendet). Prinzipiell kommen auch von Kugeln verschiedene Formkörper in Betracht (z.B. Zylinder oder Ringe) die eine den vorgenannten Durchmessern entsprechende Längstausdehnung (größte direkte Verbindung zweier auf ihrer Oberfläche befindlicher Punkte) aufweisen. Als alternative Inertmaterialien zum Steatit kommen insbesondere Aluminiumoxide, Siliciumdioxid, Thoriumdioxid, Zirkondioxid, Siliciumcarbid oder Silikate wie Magnesium- und Aluminiumsilikat in Betracht. Erst auf diese Inertschicht wird anschließend die Katalysatorschüttung aufgebracht. Das Reaktionsgas durchströmt den Reaktionsraum in entsprechender Weise von oben nach unten. Nach oben wird die Katalysatorschüttung nochmals durch eine entsprechende Inertschüttung abgedeckt.

Eine den wenigstens einen Ausgangsgasstrom zuführende Rohrleitung wird mit der wenigstens einen ersten Öffnung O1 normalerweise ebenso durch Anflanschen gasdicht verbunden, wie eine den wenigstens einen Produktgasstrom abführende Rohrleitung mit der wenigstens einen zweiten Öffnung 02.

Die vorgenannten (Rohr) Leitungen werden für einen sachgerechten und sicheren Betrieb bevorzugt mit Einrichtungen zur Kompensation von Längenausdehnungseffekten, wie sie z.B. aufgrund von Temperaturänderungen auftreten können, ausgerüstet, wobei mit Vorteil Kompensatoren eingesetzt werden, die sich durch laterale Wirkungsweise auszeichnen.

Diese in der Regel mehrschichtig ausgeführten Kompensatoren können aus dem gleichen Werkstoff wie die Rohrleitung selbst gefertigt sein. Besonders vorteilhaft sind jedoch Ausführungsformen mit (allgemein: gasdurchlässigem starrem Innenrohr und gasundurchlässiger elastischer Außenhülle (gasundurchlässigem elastischem Außenrohr)) einem das zu führende Gas berührenden inneren, vorzugsweise aus einem erfindungsgemäßen Stahl S gefertigten, Rohrteil, der zweckmäßig eine gasdurchlässige Dehnfuge aufweist, und einem außenliegenden, gasundurchlässigen, elastischen, gewellten Teil, der wenigstens teilweise aus einem insbesondere mechanisch und temperaturbelastbaren Werkstoff gefertigt ist, wie z.B. dem Werkstoff 1.4876 (Bezeichnung nach VdTÜV-Wb 434) bzw. 1.4958/1.4959 (Bezeichnung nach DIN 17459) bzw. IN-COLOY 800 H bzw. 800 HT oder Nickelbasiswerkstoff 2.4816 (alternative Bezeichnung Alloy 600) oder 2.4851 (alternative Bezeichnung Alloy 601).

Um die Verweilzeit eines zugeführten Gases im Innenraum I einer Einhüllenden E erfindungsgemäß vorteilhaft zu minimieren, ist es zweckmäßig, das Haubeninnenvolumen mittels innerhalb der Haube angebrachter Verdrängerkörper zu verkleinern.

Alternativ zu vorgenanntem Lösungsansatz empfiehlt sich die Verwendung einer konkav (ins Innere des ringförmigen Segments R) gewölbten Haube. In diesem Fall wird man die wenigstens eine erste Öffnung O1 zur Zufuhr des wenigstens einen Gasstroms in den Innenraum mit Vorteil nicht im Zentrum der oberen Haube anbringen. Vielmehr ist es in diesem Fall zweckmäßig, um den Umfang des obersten Abschnitts des ringförmigen Segments R, zweckmäßig gleichmäßig verteilt, als solche Öffnungen Fenster mit Durchtritt zum ringförmigen Innenraum I_{R} anzubringen. Von außen wird man um die Fensterleiste herum zweckmäßig einen Ringkanal anbringen, der gasdicht an die Fensterleiste anschließt (ähnlich dem Ringkanal zur Zufuhr von Salzschmelze bei einem salzbadgekühlten Rohrbündelreaktor; vgl. Ziffer 22 in DE-A 19806810, Fig. 1). Den Ausgangsgasstrom wird man dann dem Ringkanal zuführen, der diesen Gasstrom über alle Fenster gleichmäßig verteilt und durch die Fenster hindurch dem Innenraum I_{R} zuführt. Im Innenraum I_{R} können mit kontrolliertem Abstand zu den Fenstern Prallplatten angebracht sein, die den dem Innenraum I_{R} zugeführten Ausgangsgasstrom in seiner Verteilung über den Querschnitt des Innenraums I_{R} zusätzlich vergleichmäßigen.

Zum Zweck der adiabaten Durchführung einer heterogen katalysierten Dehydrierung wenigstens eines zu dehydrierenden Kohlenwasserstoffs im Innenraum I (insbesondere im Innenraum I_{R}) der Einhüllenden E, wird man auf die vom Innenraum I abgewandte Seite der Einhüllenden E (einschließlich Hauben und Zufuhr- bzw. Abfuhrleitungen) Wärmedämmmaterial (= Materialien, die die Wärmeübergangszahl für den Wärmeübergang von der Einhüllenden an die Umgebung verringern) anbringen (z.B. Glaswolle, Steinwolle und/oder Keramikwolle). Bei Bedarf können vorgenannte Dämmmaterialien zusätzlich auf die dem Innenraum zugewandte Seite der Einhüllenden E angebracht werden. Dabei können diese ihrerseits eine Trennumhüllende, z.B. aus erfindungsgemäßem Stahlblech, aufweisen. Insgesamt ist die Wärmedämmung bevorzugt so beschaffen, dass, bezogen auf den einfachen Durchgang des Reaktionsgases durch den Innenraum I, ≤ 10 %, bevorzugt ≤ 5 % und besonders bevorzugt ≤ 2 % des über den Innenraum I gemittelten Wärmeinhalts während der Ausführung eines erfindungsgemäßen Verfahrens im Innenraum I an die äußere Umgebung der Einhüllenden E abfließen. Für den Fall einer adiabat geführten konventionellen oxidativen heterogen katalysierten Dehydrierung wenigstens eines zu dehydrierenden Kohlenwasserstoffs (z.B. Propan zu Propylen) wird man durch die Einhüllende E Leitungen in den Innenraum I führen, durch die z.B. zwischen den Katalysatorhorden molekularen Sauerstoff enthaltendes Gas (z.B. Luft) zugedüst werden kann, das der exothermen katalytischen Verbrennung von molekularem Wasserstoff sowie gegebenenfalls Kohlenwasserstoff dient.

Als alternative Ausführungsform kommen für das erfindungsgemäße Verfahren aber auch Einhüllende E in Betracht, die eine Hohlkugel umschreiben. In diesen Fällen weist die Einhüllende E ein hohlkugelzonenförmiges Segment K auf, das dadurch erdacht werden kann, dass eine Hohlkugel von zwei parallelen Ebenen geschnitten wird. Zwischen den beiden Ebenen entsteht dann das hohlkugelzonenförmige Segment K und oberhalb und unterhalb der beiden Ebenen wird je eine Hohlkugelkappe abgeschnitten, die die Funktion der das Segment K nach außen abschließenden Hauben übernehmen können. Der Innenraum I_{K} des Segments K bildet wiederum den besonders relevanten (I_{R} entsprechenden) Teil des Innenraums I. Im übrigen gilt auch im Fall einer kugelförmigen Einhüllenden E das bei der zylindrischen Einhüllenden E hinsichtlich der Unterbringung von Katalysatorhorden sowie Zufuhr- und Abfuhröffnungen O1 und 02 betreffend Gesagte alles in analoger Weise. Das gleiche gilt für die Aussagen zur Wärmedämmung. Um die Einhüllende E selbst wird man generell mit Vorteil einen Tragring anbringen, der zweckmäßig auf vier Stützen gestellt die Einhüllende E hält.

Enthält der von der Einhüllenden E gebildete Innenraum I Einbauten wie Gitterroste etc., so sind diese bevorzugt gleichfalls aus erfindungsgemäßem Stahl S gefertigt. Sie können für das erfindungsgemäße Verfahren grundsätzlich aber auch z.B. aus gebrannten Tonen (Aluminiumsilicaten) oder Steatit (z.B. C 220 der Fa. CeramTec), oder sonstigen (im wesentlichen inerten) Hochtemperaturkeramikmaterialien wie Aluminiumoxiden, Siliciumdioxid, Thoriumdioxid, Zirkondioxid, Siliciumcarbid oder sonstigen Silicaten wie Magnesiumsilicat gefertigt sein.

Erfindungsgemäß vorteilhaft ist die Oberfläche der Einhüllenden E wenigstens auf ihrer den Innenraum I_{R} bzw. den Innenraum I_{K} berührenden Teilfläche (bzw. auf wenigstens 10%, bzw. wenigstens 20 %, bzw. wenigstens 30 %, bzw. wenigstens 40 %, bzw. wenigstens 50 %, bzw. wenigstens 60 %, bzw. wenigstens 70 %, bzw. wenigstens 80 %, bzw. wenigstens 90 % oder wenigstens 95 % dieser Teilfläche) in einer Schichtdicke d von wenigstens 1 mm (bzw. wenigstens 2 mm, oder wenigstens 3 mm, oder wenigstens 4 mm, oder wenigstens 5 mm, oder ≥ 5 mm bis 30 mm, oder bis zu 25 mm, oder bis zu 20 mm, oder bis zu 15 mm, oder bis zu 10 mm) aus erfindungsgemäßem Stahl S gefertigt. Vorzugsweise ist das Segment R bzw. das Segment K der Einhüllenden E zu wenigstens 80 %, oder zu wenigstens 90 %, mit Vorteil zu wenigstens 95 % und ganz besonders vorteilhaft zu 100 % ihres Gewichtes aus erfindungsgemäßem Stahl S gefertigt. Das für die Segmente R bzw. K Gesagte trifft so auch auf die Hauben und deren innere Oberflächen zu.

Wird zur Fertigung einer Einhüllenden E erfindungsgemäßer Stahl S verschweißt, so erfolgt eine solche Verschweißung erfindungsgemäß bevorzugt artgleich, d.h., Basiswerkstoff und Schweißzusatzwerkstoff sind identisch. Prinzipiell kann zu diesem Zweck aber auch tiefer schmelzender Stahl verwendet werden, der in seiner Elementzusammensetzung erfindungsgemäßem Stahl S möglichst nahe kommt. Vorzugsweise erfolgt das Schweißen unter Inertgasatmosphäre (besonders bevorzugt unter Argon und/oder Helium).

Geeignete Schmelzschweißverfahren sind das Lichtbogenschweißen mit Stabelektroden sowie das Schutzgasschweißen (vor allem nach dem WIG-Verfahren). Als Schutzgas wird bevorzugt ein stickstoffhaltiges Schutzgas verwendet. Als Schweißelektroden können die Schweißstäbe Thermanit D (W 22 12 H) oder Thermanit C (W 25 20 Mn) oder Sandvik 22 12 HT verwendet werden.

Abschließend sei noch vermerkt, dass die erfindungsgemäßen Stähle S auch hinsichtlich ihrer vergleichsweise beschränkten katalysierenden Wirkung hinsichtlich einer Vollverbrennung von zu dehydrierendem und/oder dehydriertem Kohlenwasserstoff im Beisein von molekularem Sauerstoff zu befriedigen vermögen.

### Beispiele und Vergleichsbeispiele

### I. Allgemeine Versuchsbeschreibung

1. Gestaltung der Reaktionsrohre
   Die Geometrie der Reaktionsrohre war:
   0,55 m lang
   21,34 bis 22 mm Außendurchmesser (A)
   2 bis 2,77 mm Wanddicke (W)
   Das jeweilige Reaktionsrohr war auf seiner gesamten Länge mit inerten Kugeln aus Steatit C 220 der Fa. CeramTec gefüllt. Der Kugeldurchmesser betrug im wesentlichen gleichförmig verteilt 1,5 mm bis 2,5 mm.
2. Als Werkstoff für das Reaktionsrohr wurden 5 unterschiedliche Materialien verwendet.
   - Material 1 (M1):: Edelstahl der DIN Werkstoffnummer 1.4841 (A = 22 mm, W = 2 mm),
   - Material 2 (M2):: Edelstahl der DIN Werkstoffnummer 1.4541, der nachfolgende Elementzusammensetzung aufwies (A = 22 mm, W = 2 mm):
   17,31 Gew.-% Cr,
   10,05 Gew.-% Ni,
   0,51 Gew.-% Si,
   0,017 Gew.-% N,
   0,042 Gew.-% C,
   1,17 Gew.-% Mn,
   0,025 Gew.-% P,
   < 0,0005 Gew.-% S,
   0,29 Gew.-% Ti sowie im übrigen Fe und herstellungsbedingte Verunreinigungen, wobei die Prozentangaben jeweils auf das Gesamtgewicht bezogen sind (Prüfzeugnis von Sterling Tubes).
   - Material 3 (M3):: Edelstahl der DIN Werkstoffnummer 1.4541, jedoch auf seiner
   dem Reaktionsraum zugewandten Seite oberflächenalonisiert (A = 22 mm, W = 2 mm).
   - Material 4 (M4):: Gebranntes Aluminiumnidrid (A = 22 mm, W = 2 mm)
   - Material 5 (M5):: Erfindungsgemäßer Edelstahl der nachfolgenden Elementzusammensetzung (A = 21,34 mm, W = 2,77 mm):
   20,87 Gew.-% Cr,
   10,78 Gew.-% Ni,
   1,54 Gew.-% Si,
   0,161 Gew.-% N,
   0,082 Gew.-% C,
   0,75 Gew.-% Mn,
   0,02 Gew.-% P,
   0,0026 Gew.-% S,
   0,05 Gew.-% Ce, und
   im übrigen Fe und herstellungsbedingte Verunreinigungen, wobei die Prozentangaben jeweils auf das Gesamtgewicht bezogen sind.
3. Die verschiedenen Reaktionsrohre wurden mit nachfolgenden Ausgangsgasströmen beschickt, wie sie in ihrer Zusammensetzung für eine erfindungsgemäße heterogen katalysierte Dehydrierung von Propan zu Propylen typisch sind:
   - Feed A:: 31,7 Vol.-% Propan,
   51,0 Vol.-% N₂,
   3,09 Vol.-% O₂,
   6,33 Vol.-% H₂, und
   7,88 Vol.-% H₂O
   - Feed B:: 33,8 Vol.-% Propan,
   54,5 Vol.-% N₂,
   3,3 Vol.-% O₂, und
   8,4 Vol.-% H₂O.
   - Feed C:: 33,8 Vol.-% Propan,
   57,8 Vol.-% N₂ und
   8,4 Vol.-% H₂O.

   Die gewählte Propanbelastung des Festbetts aus inerten Steatitkugeln wurde in allen Fällen zu 20 Nl/l•h gewählt.
4. Das Reaktionsrohr war jeweils in einem Strahlungsofen untergebracht (elektrisch beheizter keramischer Körper, mit hohlzylindrischer Führung zur Aufnahme des Reaktionsrohres mit einem Spaltabstand von 0,2 cm zur Reaktionsrohraußenwand).
5. Das jeweilige Reaktionsrohr wurde wie beschrieben vom jeweiligen Ausgangsgasstrom (dieser wies jeweils eine Eingangstemperatur von 200°C auf) durchströmt. Gleichzeitig wurde die Temperatur T^{A} der Außenwand des Reaktionsrohres so erhöht, dass sich die maximale Temperatur T^{M} im Reaktionsrohr im wesentlichen linear mit einem Gradienten von 10°C/h von 400°C auf 700°C erhöhte (dies simuliert die Kompensation eines im kontinuierlichen Betrieb deaktivierenden Katalysatorbettes durch Erhöhung der Reaktionstemperatur).

Anschließend wurde die Regenerierung eines Dehydrierkatalysatorbetts simuliert. Dazu wurde das Reaktionsrohr zunächst mit 420 Nml/min N₂ der Eingangstemperatur 200°C durchströmt und dabei die Temperatur T^{M} auf 700°C gehalten.

Unter Beibehalt der Temperatur T^{M} = 700°C wurde folgendes Gasdurchströmprogramm durchlaufen:
- während 60 min Magerluft (Gemisch aus Luft (85,4 Nml/min) und N₂ (341,6 Nml/min));

dann - während 60 min 417 Nml/min Luft;
dann - während 15 min 417 Nml/min N₂;
dann - während 60 min 168 Nml/min H₂.

Danach wurde das jeweilige Reaktionsrohr, vom jeweiligen Feed durchströmt, mit einem T^{M}- Gradienten von 10°C/h im wesentlichen linear von T^{M} = 700°C auf T^{M} = 400°C gebracht.

Dabei wurden in Abhängigkeit vom verwendeten Feed sowie vom für das Reaktionsrohr verwendeten Material bei den Temperaturen T^{A} von 500°C, 600°C, 650°C und 700°C die nachfolgenden Werte N^{P} (%) ermittelt (am Ausgang des Reaktionsrohres wurde die Zusammensetzung des austretenden Gasstromes kontinuierlich analysiert (gaschromatographisch); ermittelt wurde die Nebenproduktgesamtmenge N^{P} an den von Propan verschiedenen Kohlenwasserstoffen Methan, Ethan und Ethylen, ausgedrückt als Gesamtkohlenstoffgehalt dieser Kohlenwasserstoffnebenproduktgesamtmenge, bezogen auf die dem Reaktionsrohr als Propan zugeführte Gesamtkohlenstoffmenge (in %)):

**Feed A**

| | M1 | M2 | M3 | M4 | M5 |
|---|---|---|---|---|---|
| 550°C | 0,031 | 0,029 | 0,023 | 0,013 | 0,026 |
| 600°C | 0,117 | 0,037 | 0,036 | 0,026 | 0,066 |
| 650°C | 0,939 | 0,683 | 0,539 | 0,197 | 0,243 |
| 700°C | 7,320 | 2,840 | 4,841 | 2,010 | 1,520 |

**Feed B**

| | M1 | M2 | M3 | M4 | M5 |
|---|---|---|---|---|---|
| 550°C | 0,033 | 0,010 | 0,006 | 0,003 | 0,052 |
| 600°C | 0,149 | 0,177 | 0,023 | 0,009 | 0,076 |
| 650°C | 0,865 | 0,732 | 0,257 | 0,089 | 0,235 |
| 700°C | 4,735 | 2,650 | 3,201 | 1,273 | 1,345 |

**Feed C**

| | M1 | M2 | M3 | M4 | M5 |
|---|---|---|---|---|---|
| 550°C | 0,026 | 0,022 | 0,011 | 0,023 | 0,007 |
| 600°C | 0,176 | 0,344 | 0,116 | 0,052 | 0,062 |
| 650°C | 1,540 | 1,240 | 0,981 | 0,427 | 0,555 |
| 700°C | 6,190 | 3,190 | 4,681 | 2,650 | 3,147 |

US Provisional Patent Application No. 60/751,973, eingereicht am 21.12.2005, ist in die vorliegende Patentanmeldung durch Literaturhinweis eingefügt.

Im Hinblick auf die obengenannten Lehren sind zahlreiche Änderungen und Abweichungen von der vorliegenden Erfindung möglich.

Man kann deshalb davon ausgehen, dass die Erfindung, im Rahmen der beigefügten Ansprüche, anders als hierin spezifisch beschrieben, ausgeführt werden kann.

## Patentansprüche

1. Verfahren der kontinuierlichen heterogen katalysierten partiellen Dehydrierung wenigstens eines zu dehydrierenden Kohlenwasserstoffs in der Gasphase, umfassend eine Verfahrensweise, bei der man einem Reaktionsraum, der von einer den Reaktionsraum berührenden Einhüllenden umschlossen ist, die wenigstens eine erste Öffnung zur Zufuhr wenigstens eines Ausgangsgasstroms in den Reaktionsraum und wenigstens eine zweite Öffnung zur Entnahme wenigstens eine Produktgasstroms aus dem Reaktionsraum aufweist,
- wenigstens einen, wenigstens einen zu dehydrierenden Kohlenwasserstoff enthaltenden, Ausgangsgasstrom kontinuierlich zuführt,
- im Reaktionsraum den wenigstens einen zu dehydrierenden Kohlenwasserstoff durch wenigstens ein im Reaktionsraum befindliches Katalysatorbett führt, und unter Erzeugung eines den wenigstens einen dehydrierten Kohlenwasserstoff, nicht umgesetzten zu dehydrierenden Kohlenwasserstoff sowie molekularen Wasserstoff und/oder Wasserdampf enthaltenden Produktgases oxidativ oder nicht oxidativ zu wenigstens einem dehydrierten Kohlenwasserstoff partiell dehydriert, und
- dem Reaktionsraum wenigstens einen Produktgasstrom kontinuierlich entnimmt,
**dadurch gekennzeichnet, dass**
die Oberfläche der Einhüllenden auf ihrer den Reaktionsraum berührenden Seite wenigstens teilweise in einer Schichtdicke d von wenigstens 1 mm aus einem Stahl S gefertigt ist, der die nachfolgende Elementzusammensetzung aufweist:
18 bis 30 Gew.-% Cr,
9 bis 36 Gew.-% Ni,
1 bis 3 Gew.-% Si,
0,1 bis 0,3 Gew.-% N,
≥ 0 bis 0,15 Gew.-% C,
≥ 0 bis 4 Gew.-% Mn,
≥ 0 bis 4 Gew.-% Al,
≥ 0 bis 0,05 Gew.-% P,
≥ 0 bis 0,05 Gew.-% S, und
≥ 0 bis 0,1 Gew.-% eines oder mehrere seltene Erdmetalle, und
im übrigen Fe und herstellungsbedingte Verunreinigungen, wobei die Prozentangaben jeweils auf das Gesamtgewicht bezogen sind.

2. Verfahren nach Anspruch 1, **dadurch gekennzeichnet, dass** der Stahl S die nachfolgende Elementzusammensetzung aufweist:
20 bis 25 Gew.-% Cr,
9 bis 20 Gew.-% Ni,
1,4 bis 2,5 Gew.-% Si,
0,1 bis 0,3 Gew.-% N,
0,03 bis 0,15 Gew.-% C,
≥ 0 bis 3 Gew.-% Mn,
≥ 0 bis 4 Gew.-% Al,
≥ 0 bis 0,05 Gew.-% P,
≥ 0 bis 0,05 Gew.-% S, und
≥ 0 bis 0,1 Gew.-% eines oder mehrere seltene Erdmetalle, und
im übrigen Fe und herstellungsbedingte Verunreinigungen, wobei die Prozentangaben jeweils auf das Gesamtgewicht bezogen sind.

3. Verfahren nach Anspruch 1, **dadurch gekennzeichnet, dass** der Stahl S die nachfolgende Elementzusammensetzung aufweist:
20 bis 22 Gew.-% Cr,
10 bis 12 Gew.-% Ni,
1,4 bis 2,5 Gew.-% Si,
0,12 bis 0,2 Gew.-% N,
0,05 bis 0,12 Gew.-% C,
≥ 0 bis 1 Gew.-% Mn,
≥ 0 bis 2 Gew.-% Al,
≥ 0 bis 0,045 Gew.-% P,
≥ 0 bis 0,015 Gew.-% S, und
≥ 0,03 bis 0,08 Gew.-% Ce oder Ce und eines oder mehrere seltene Erdmetalle, und
im übrigen Fe und herstellungsbedingte Verunreinigungen, wobei die Prozentangaben jeweils auf das Gesamtgewicht bezogen sind.

4. Verfahren nach einem der Ansprüche 1 bis 3, **dadurch gekennzeichnet, dass** der zu dehydrierende Kohlenwasserstoff ein C₂- bis C₁₆-Alkan ist.

5. Verfahren nach einem der Ansprüche 1 bis 3, **dadurch gekennzeichnet, dass** der zu dehydrierende Kohlenwasserstoff wenigstens ein Kohlenwasserstoff aus der Gruppe umfassend Ethan, Propan, n-Butan, iso-Butan, n-Pentan, iso-Pentan, n-Hexan, n-Heptan, n-Octan, n-Nonan, n-Decan, n-Undecan, n-Dodecan, n-Tridecan, n-Tetradecan, n-Pentadecan und n-Hexadecan ist.

6. Verfahren nach einem der Ansprüche 1 bis 3, **dadurch gekennzeichnet, dass** der zu dehydrierende Kohlenwasserstoff Ethan, Propan, n-Butan und/oder iso-Butan ist.

7. Verfahren nach einem der Ansprüche 1 bis 3, **dadurch gekennzeichnet, dass** der zu dehydrierende Kohlenwasserstoff Propan und der dehydrierte Kohlenwasserstoff Propylen ist.

8. Verfahren nach einem der Ansprüche 1 bis 7, **dadurch gekennzeichnet, dass** der Ausgangsgasstrom Wasserdampf enthält.

9. Verfahren nach einem der Ansprüche 1 bis 8, **dadurch gekennzeichnet, dass** der Ausgangsgasstrom molekularen Sauerstoff enthält.

10. Verfahren nach einem der Ansprüche 1 bis 9, **dadurch gekennzeichnet, dass** das Katalysatorbett ein Katalysatorfestbett ist.

11. Verfahren nach einem der Ansprüche 1 bis 10, **dadurch gekennzeichnet, dass** die Oberfläche der Einhüllenden auf ihrer den Reaktionsraum berührenden Seite zu wenigstens 10 % ihrer Gesamtfläche in einer Schichtdicke d von wenigstens 1 mm aus einem Stahl S gefertigt ist.

12. Verfahren nach einem der Ansprüche 1 bis 10, **dadurch gekennzeichnet, dass** die Oberfläche der Einhüllenden auf ihrer den Reaktionsraum berührenden Seite zu wenigstens 50% ihrer Gesamtfläche in einer Schichtdicke d von wenigstens 1 mm aus einem Stahl S gefertigt ist.

13. Verfahren nach einem der Ansprüche 1 bis 12, **dadurch gekennzeichnet, dass** d wenigstens 3 mm beträgt.

14. Verfahren nach einem der Ansprüche 1 bis 12, **dadurch gekennzeichnet, dass** d wenigstens 5 mm beträgt.

15. Verfahren nach einem der Ansprüche 1 bis 14, **dadurch gekennzeichnet, dass** die Einhüllende zu wenigstens 80 % ihres Gewichtes aus einem Stahl S gefertigt ist.

16. Verfahren nach einem der Ansprüche 1 bis 15, **dadurch gekennzeichnet, dass** die Einhüllende in ihrer Gesamtheit aus einem Stahl S gefertigt ist.

17. Verfahren nach einem der Ansprüche 1 bis 16, **dadurch gekennzeichnet, dass** die heterogen katalysierte partielle Dehydrierung eine nicht oxidative Dehydrierung ist.

18. Verfahren nach einem der Ansprüche 1 bis 16, **dadurch gekennzeichnet, dass** die heterogen katalysierte partielle Dehydrierung eine oxidative Dehydrierung ist.

19. Verfahren nach einem der Ansprüche 1 bis 16, **dadurch gekennzeichnet, dass** die heterogen katalysierte partielle Dehydrierung eine heterogen katalysierte Oxidehydrierung ist.

20. Verfahren nach einem der Ansprüche 1 bis 16, **dadurch gekennzeichnet, dass** die heterogen katalysierte partielle Dehydrierung eine adiabat ausgeführte konventionelle heterogen katalysierte Dehydrierung ist.

21. Verfahren nach einem der Ansprüche 1 bis 16, **dadurch gekennzeichnet, dass** die heterogen katalysierte partielle Dehydrierung eine konventionelle heterogen katalysierte partielle Dehydrierung und der Reaktionsraum ein Hordenreaktionsraum ist.

22. Verfahren nach Anspruch 21, **dadurch gekennzeichnet, dass** die konventionelle heterogen katalysierte partielle Dehydrierung eine oxidative konventionelle heterogen katalysierte partielle Dehydrierung ist.

23. Verfahren nach Anspruch 22, **dadurch gekennzeichnet, dass** es adiabat ausgeführt wird.

24. Verfahren nach einem der Ansprüche 1 bis 23, **dadurch gekennzeichnet, dass** der dem Reaktionsraum zugeführte Ausgangsgasstrom enthält:
Propylen ≥ 0 bis 20 Vol.-%,
Acrolein ≥ 0 bis 1 Vol.-%,
Acrylsäure ≥ 0 bis 0,25 Vol.-%,
COₓ ≥ 0 bis 20 Vol.-%,
Propan 5 bis 50 Vol.-%,
Stickstoff 20 bis 80 Vol.-%,
Sauerstoff ≥ 0 bis 5 Vol.-%,
H₂O ≥ 0 bis 20 Vol.-% und
H₂ ≥ 0 bis 10 Vol.-%.

25. Verfahren nach einem der Ansprüche 1 bis 24, **dadurch gekennzeichnet, dass** man den aus dem Reaktionsraum entnommenen Produktgasstrom als solchen oder nach Abtrennung wenigstens einer Teilmenge seiner vom dehydrierten Kohlenwasserstoff und vom zu dehydrierenden Kohlenwasserstoff verschiedenen Bestandteile zur Beschickung wenigstens eines Oxidationsreaktors verwendet und in diesem den darin enthaltenen dehydrierten Kohlenwasserstoff einer selektiven heterogen katalysierten partiellen Gasphasenoxidation mit molekularem Sauerstoff zu einem das Partialoxidationsprodukt enthaltenden Produktgasgemisch B unterwirft.

26. Verfahren nach Anspruch 25, **dadurch gekennzeichnet, dass** der zu dehydrierende Kohlenwasserstoff Propan, der dehydrierte Kohlenwasserstoff Propylen und das Partialoxidationsprodukt Acrolein, Acrylsäure oder deren Gemisch ist.

27. Verfahren nach Anspruch 25, **dadurch gekennzeichnet, dass** man in einer Trennzone B der selektiven heterogen katalysierten partiellen Gasphasenoxidation nachfolgend aus dem Produktgasgemisch B Partialoxidationsprodukt abtrennt und von dem dabei verbleibenden, nicht umgesetzten zu dehydrierenden Kohlenwasserstoff, molekularen Sauerstoff sowie gegebenenfalls nicht umgesetzten dehydrierten Kohlenwasserstoff enthaltenden Restgas wenigstens eine nicht umgesetzten zu dehydrierenden Kohlenwasserstoff enthaltende Teilmenge als Partialoxidationskreisgas in das Verfahren der heterogen katalysierten partiellen Dehydrierung des zu dehydrierenden Kohlenwasserstoffs rückführt.

28. Verfahren nach Anspruch 27, **dadurch gekennzeichnet, dass** man das Partialoxidationsprodukt in der Trennzone B vom Produktgasgemisch B durch Überführen in die kondensierte Phase abtrennt.

29. Verfahren nach Anspruch 28, **dadurch gekennzeichnet, dass** das Partialoxidationsprodukt Acrylsäure ist und die Überführung in die kondensierte Phase durch absorptive und/oder kondensative Maßnahmen erfolgt.

30. Verfahren nach Anspruch 29, **dadurch gekennzeichnet, dass** unter Anwendung wenigstens eines thermischen Trennverfahrens eine Abtrennung der Acrylsäure aus der kondensierten Phase vorgenommen wird.

31. Verfahren nach Anspruch 30, **dadurch gekennzeichnet, dass** das wenigstens eine thermische Trennverfahren eine kristallisative Abtrennung von Acrylsäure aus flüssiger Phase umfasst.

32. Verfahren nach Anspruch 31, **dadurch gekennzeichnet, dass** die kristallisative Abtrennung eine Suspensionskristallisation ist.

33. Verfahren nach Anspruch 30, **dadurch gekennzeichnet, dass** sich an die Abtrennung der Acrylsäure ein Verfahren der radikalischen Polymerisation anschließt, in welchem abgetrennte Acrylsäure zur Herstellung von Polymerisaten radikalisch einpolymerisiert wird.

34. Verfahren nach Anspruch 30, **dadurch gekennzeichnet, dass** sich an die Abtrennung der Acrylsäure ein Verfahren zur Herstellung von Acrylsäureestern anschließt, in welchem abgetrennte Acrylsäure mit einem Alkohol verestert wird.

35. Verfahren nach Anspruch 34, **dadurch gekennzeichnet, dass** sich an das Verfahren zur Herstellung eines Acrylsäureester ein Verfahren der radikalischen Polymerisation anschließt, in welchem so hergestellter Acrylsäureester einpolymerisiert wird.

36. Eine einen Innenraum I umschließende Einhüllende E mit wenigstens einer ersten Öffnung O1 zur Zufuhr wenigstens eines Gasstroms S in den Innenraum I und wenigstens einer zweiten Öffnung 02 zur Entnahme eines dem Innenraum I zuvor über die wenigstens eine erste Öffnung O1 zugeführten Gasstroms S aus dem Innenraum I, wobei die Oberfläche der Einhüllenden E auf ihrer den Innenraum I berührenden Seite wenigstens teilweise in einer Schichtdicke d von wenigstens 1 mm aus einem Stahl S gefertigt ist, der die nachfolgende Elementzusammensetzung aufweist:
18 bis 30 Gew.-% Cr,
9 bis 36 Gew.-% Ni,
1 bis 3 Gew.-% Si,
0,1 bis 0,3 Gew.-% N,
≥ 0 bis 0,15 Gew.-% C,
≥ 0 bis 4 Gew.-% Mn,
≥ 0 bis 4 Gew.-% Al,
≥ 0 bis 0,05 Gew.-% P,
≥ 0 bis 0,05 Gew.-% S, und
≥ 0 bis 0,1 Gew.-% eines oder mehrere seltene Erdmetalle, und
im übrigen Fe und herstellungsbedingte Verunreinigungen, wobei die Prozentangaben jeweils auf das Gesamtgewicht bezogen sind.

37. Eine Einhüllende E nach Anspruch 36, deren Innenraum I wenigstens einen Dehydrierkatalysator enthält.

38. Eine Einhüllende E nach Anspruch 36 oder 37, deren Innenraum I wenigstens einen Rost enthält.

39. Eine Einhüllende E nach einem der Ansprüche 36 bis 38, die ein ringförmiges Segment R aufweist.

40. Eine Einhüllende E nach Anspruch 39, wobei das Verhältnis V₁=D : A, gebildet aus der Hälfte D der Differenz zwischen dem Außendurchmesser A und dem Innendurchmesser des ringförmigen Segments R, 1 : 10 bis 1 : 1000 beträgt.

41. Eine Einhüllende E nach Anspruch 40, wobei V₁ 1 : 40 bis 1 : 500 beträgt.

42. Eine Einhüllende E nach einem der Ansprüche 39 bis 41, wobei das Verhältnis V₂=H : A, gebildet aus dem Abstand H der beiden das ringförmige Segment R begrenzenden parallelen kreisförmigen Ebenen und dem Außendurchmesser A des ringförmigen Segments > 1 beträgt.

43. Eine Einhüllende E nach einem der Ansprüche 39 bis 41, wobei das Verhältnis V₂=H : A, gebildet aus dem Abstand H der beiden das ringförmige Segment R begrenzenden parallelen kreisförmigen Ebenen und dem Außendurchmesser des ringförmigen Segments ≤ 1 beträgt.

44. Eine Einhüllende E nach einem der Ansprüche 36 bis 38, die ein hohlkugelzonenförmiges Segment K aufweist.

45. Eine Einhüllende E nach einem der Ansprüche 36 bis 44, die auf ihrer vom Innenraum I abgewandten Seite Wärmedämmmaterial aufgebracht aufweist.

46. Verfahren der heterogen katalysierten partiellen Dehydrierung eines Kohlenwasserstoffs, **dadurch gekennzeichnet, dass** man es im Innenraum I einer Einhüllenden E gemäß einem der Ansprüche 36 bis 45 durchführt.

47. Verwendung einer Einhüllenden E gemäß einem der Ansprüche 36 bis 45 zur Durchführung einer heterogen katalysierten partiellen Dehydrierung eines Kohlenwasserstoffs.

48. Verfahren nach einem der Ansprüche 1 bis 35, **dadurch gekennzeichnet, dass** der Stahl S auf seiner den Reaktionsraum berührenden Seite alonisiert, alitiert und/oder aluminiert ist.

49. Einhüllende E nach einem der Ansprüche 36 bis 45, wobei der Stahl S auf der den Innenraum I berührenden Seite alonisiert, alitiert und/oder aluminiert ist.

## Claims

1. A process for continuous heterogeneously catalyzed partial dehydrogenation of at least one hydrocarbon to be dehydrogenated in the gas phase, comprising a procedure in which a reaction chamber which is enclosed by a shell which is in contact with the reaction chamber and has at least one first orifice for feeding at least one starting gas stream into the reaction chamber and at least one second orifice for withdrawing at least one product gas stream from the reaction chamber,
- at least one starting gas stream comprising at least one hydrocarbon to be dehydrogenated is fed continuously,
- in the reaction chamber, the at least one hydrocarbon to be dehydrogenated is passed through at least one catalyst bed disposed in the reaction chamber and, with generation of a product gas comprising the at least one dehydrogenated hydrocarbon, unconverted hydrocarbon to be dehydrogenated and molecular hydrogen and/or steam, is dehydrogenated partially in an oxidative or nonoxidative manner to at least one dehydrogenated hydrocarbon, and
- at least one product gas stream is withdrawn continuously from the reaction chamber, wherein
the surface of the shell, on its side in contact with the reaction chamber, is manufactured at least partly, in a layer thickness d of at least 1 mm, from a steel S which has the following elemental composition:
from 18 to 30% by weight of Cr,
from 9 to 36% by weight of Ni,
from 1 to 3% by weight of Si,
from 0.1 to 0.3% by weight of N,
from ≥ 0 to 0.15% by weight ofC,
from ≥ 0 to 4% by weight of Mn,
from ≥ 0 to 4% by weight of Al,
from ≥ 0 to 0.05% by weight ofP,
from ≥ 0 to 0.05% by weight ofS, and
from ≥ 0 to 0.1% by weight of one or more rare earth metals, and,
apart from these, Fe and impurities resulting from production, the percentages each being based on the total weight.

2. The process according to claim 1, wherein the steel S has the following elemental composition:
from 20 to 25% by weight of Cr,
from 9 to 20% by weight of Ni,
from 1.4 to 2.5% by weight of Si,
from 0.1 to 0.3% by weight of N,
from 0.03 to 0.15% by weight of C,
from ≥ 0 to 3% by weight of Mn,
from ≥ 0 to 4% by weight of Al,
from ≥ 0 to 0.05% by weight ofP,
from ≥ 0 to 0.05% by weight ofS, and
from ≥ 0 to 0.1% by weight of one or more rare earth metals, and,
apart from these, Fe and impurities resulting from production, the percentages each being based on the total weight.

3. The process according to claim 1, wherein the steel S has the following elemental composition:
from 20 to 22% by weight of Cr,
from 10 to 12% by weight of Ni,
from 1.4 to 2.5% by weight of Si,
from 0.12 to 0.2% by weight ofN,
from 0.05 to 0.12% by weight of C,
from ≥ 0 to 1% by weight of Mn,
from ≥ 0 to 2% by weight of Al,
from ≥ 0 to 0.045% by weight of P,
from ≥ 0 to 0.015% by weight of S, and
from ≥ 0.03 to 0.08% by weight of Ce or Ce and one or more rare earth metals, and,
apart from these, Fe and impurities resulting from production, the percentages each being based on the total weight.

4. The process according to any of claims 1 to 3, wherein the hydrocarbon to be dehydrogenated is a C₂- to C₁₆-alkane.

5. The process according to any of claims 1 to 3, wherein the hydrocarbon to be dehydrogenated is at least one hydrocarbon from the group comprising ethane, propane, n-butane, isobutane, n-pentane, isopentane, n-hexane, n-heptane, n-octane, n-nonane, n-decane, n-undecane, n-dodecane, n-tridecane, n-tetradecane, n-pentadecane and n-hexadecane.

6. The process according to any of claims 1 to 3, wherein the hydrocarbon to be dehydrogenated is ethane, propane, n-butane and/or isobutane.

7. The process according to any of claims 1 to 3, wherein the hydrocarbon to be dehydrogenated is propane and the dehydrogenated hydrocarbon is propylene.

8. The process according to any of claims 1 to 7, wherein the starting gas stream comprises steam.

9. The process according to any of claims 1 to 8, wherein the starting gas stream comprises molecular oxygen.

10. The process according to any of claims 1 to 9, wherein the catalyst bed is a fixed catalyst bed.

11. The process according to any of claims 1 to 10, wherein the surface of the shell, on its side in contact with the reaction chamber, is manufactured to an extent of at least 10% of its total surface area from a steel S in a layer thickness d of at least 1 mm.

12. The process according to any of claims 1 to 10, wherein the surface of the shell, on its side in contact with the reaction chamber, is manufactured to an extent of at least 50% of its total surface area from a steel S in a layer thickness d of at least 1 mm.

13. The process according to any of claims 1 to 12, wherein d is at least 3 mm.

14. The process according to any of claims 1 to 12, wherein d is at least 5 mm.

15. The process according to any of claims 1 to 14, wherein the shell is manufactured to an extent of at least 80% of its weight from a steel S.

16. The process according to any of claims 1 to 15, wherein the shell is manufactured in its entirety from a steel S.

17. The process according to any of claims 1 to 16, wherein the heterogeneously catalyzed partial dehydrogenation is a nonoxidative dehydrogenation.

18. The process according to any of claims 1 to 16, wherein the heterogeneously catalyzed partial dehydrogenation is an oxidative dehydrogenation.

19. The process according to any of claims 1 to 16, wherein the heterogeneously catalyzed partial dehydrogenation is a heterogeneously catalyzed oxydehydrogenation.

20. The process according to any of claims 1 to 16, wherein the heterogeneously catalyzed partial dehydrogenation is an adiabatic conventional heterogeneously catalyzed dehydrogenation.

21. The process according to any of claims 1 to 16, wherein the heterogeneously catalyzed partial dehydrogenation is a conventional heterogeneously catalyzed partial dehydrogenation and the reaction chamber is a tray reaction chamber.

22. The process according to claim 21, wherein the conventional heterogeneously catalyzed partial dehydrogenation is an oxidative conventional heterogeneously catalyzed partial dehydrogenation.

23. The process according to claim 22, which is performed adiabatically.

24. The process according to any of claims 1 to 23, wherein the starting gas stream fed to the reaction chamber comprises:
from ≥ 0 to 20% by volume of propylene,
from ≥ 0 to 1% by volume of acrolein,
from ≥ 0 to 0.25% by volume of acrylic acid,
from ≥ 0 to 20% by volume of COₓ,
from 5 to 50% by volume of propane,
from 20 to 80% by volume of nitrogen,
from ≥ 0 to 5% by volume of oxygen,
from ≥ 0 to 20% by volume of H₂O and
from ≥ 0 to 10% by volume of H₂.

25. The process according to any of claims 1 to 24, wherein the product gas stream withdrawn from the reaction chamber is used as such or after removal of at least a portion of its constituents other than the dehydrogenated hydrocarbon and the hydrocarbon to be dehydrogenated to charge at least one oxidation reactor, and the dehydrogenated hydrocarbon present therein is subjected in this oxidation reactor to a selective heterogeneously catalyzed partial gas phase oxidation with molecular oxygen to give a product gas mixture B comprising the partial oxidation product.

26. The process according to claim 25, wherein the hydrocarbon to be dehydrogenated is propane, the dehydrogenated hydrocarbon is propylene and the partial oxidation product is acrolein, acrylic acid or a mixture thereof.

27. The process according to claim 25, wherein, in a separation zone B of the selective heterogeneously catalyzed partial gas phase oxidation, partial oxidation product is subsequently removed from the product gas mixture B and, from the remaining residual gas comprising unconverted hydrocarbon to be dehydrogenated, molecular oxygen and any unconverted dehydrogenated hydrocarbon, at least a portion comprising unconverted hydrocarbon to be dehydrogenated is recycled as partial oxidation cycle gas into the process for heterogeneously catalyzed partial dehydrogenation of the hydrocarbon to be dehydrogenated.

28. The process according to claim 27, wherein the partial oxidation product, in separation zone B, is removed from product gas mixture B by conversion to the condensed phase.

29. The process according to claim 28, wherein the partial oxidation product is acrylic acid and the conversion to the condensed phase is effected by absorptive and/or condensative measures.

30. The process according to claim 29, wherein a removal of acrylic acid from the condensed phase is undertaken using at least one thermal separation process.

31. The process according to claim 30, wherein the at least one thermal separation process comprises a crystallizative removal of acrylic acid from the liquid phase.

32. The process according to claim 31, wherein the crystallizative removal is a suspension crystallization.

33. The process according to claim 30, wherein the removal of acrylic acid is followed by a process for free-radical polymerization in which acrylic acid removed is free-radically polymerized to prepare polymers.

34. The process according to claim 30, wherein the removal of acrylic acid is followed by a process for preparing acrylic esters in which acrylic acid removed is esterified with an alcohol.

35. The process according to claim 34, wherein the process for preparing an acrylic ester is followed by a process for free-radical polymerization in which acrylic ester thus prepared is polymerized.

36. A shell E which encloses an interior I and has at least one first orifice O1 for feeding at least one gas stream S into the interior I and at least one second orifice 02 for withdrawing a gas stream S fed to the interior I beforehand via the at least one first orifice O1 from the interior I, the surface of the shell E, on its side in contact with the interior I, being manufactured at least partly, in a layer thickness d of at least 1 mm, from a steel S which has the following elemental composition:
from 18 to 30% by weight of Cr,
from 9 to 36% by weight of Ni,
from 1 to 3% by weight of Si,
from 0.1 to 0.3% by weight of N,
from ≥ 0 to 0.15% by weight ofC,
from ≥ 0 to 4% by weight of Mn,
from ≥ 0 to 4% by weight of Al,
from ≥ 0 to 0.05% by weight ofP,
from ≥ 0 to 0.05% by weight ofS, and
from ≥ 0 to 0.1% by weight of one or more rare earth metals, and,
apart from these, Fe and impurities resulting from production, the percentages each being based on the total weight.

37. A shell E according to claim 36 whose interior I comprises at least one dehydrogenation catalyst.

38. A shell E according to claim 36 or 37 whose interior I comprises at least one grid.

39. A shell E according to any of claims 36 to 38 which has an annular segment R.

40. A shell E according to claim 39 where the ratio of V₁=D : A, formed from half D of the difference between the external diameter A and the internal diameter of the annular segment R, is from 1 : 10 to 1 : 1000.

41. A shell E according to claim 40 where V₁ is from 1 : 40 to 1 : 500.

42. A shell E according to any of claims 39 to 41 where the ratio V₂=H : A, formed from the separation H of the two parallel circular planes delimiting the annular segment R and the external diameter of the annular segment is > 1.

43. A shell E according to any of claims 39 to 41 where the ratio V₂=H : A, formed from the separation H of the two parallel circular planes delimiting the annular segment R and the external diameter of the annular segment is ≤ 1.

44. A shell E according to any of claims 36 to 38 which has a hollow spherical zone segment K.

45. A shell E according to any of claims 36 to 44 which has thermal insulation material on its side facing away from the interior I.

46. A process for heterogeneously catalyzed partial dehydrogenation of a hydrocarbon, which is performed in the interior I of a shell E according to any of claims 36 to 45.

47. The use of a shell E according to any of claims 36 to 45 for carrying out a heterogeneously catalyzed partial dehydrogenation of a hydrocarbon.

48. The process according to any of claims 1 to 35, wherein the steel S has been alonized, alitized and/or aluminized on its side in contact with the reaction chamber.

49. The shell E according to any of claims 36 to 45 where the steel S has been alonized, alitized and/or aluminized on the side in contact with the interior I.

## Revendications

1. Procédé pour la déshydrogénation partielle continue catalysée par voie hétérogène d'au moins un hydrocarbure à déshydrogéner en phase gazeuse, comprenant un mode opératoire, une chambre de réaction, qui est entourée par une gaine en contact avec la chambre de réaction et qui présente au moins une première ouverture pour l'alimentation en au moins un flux gazeux initial dans la chambre de réaction et au moins une deuxième ouverture pour le prélèvement d'au moins un flux gazeux produit de la chambre de réaction,
- étant alimentée en continu en au moins un flux gazeux initial, contenant au moins un hydrocarbure à déshydrogéner,
- on guide dans la chambre de réaction ledit au moins un hydrocarbure à déshydrogéner au travers d'au moins un lit catalytique se trouvant dans la chambre de réaction et on déshydrogène partiellement, de manière oxydante ou non oxydante, en au moins un hydrocarbure déshydrogéné, en générant un gaz produit contenant ledit au moins un hydrocarbure déshydrogéné, de l'hydrocarbure à déshydrogéner non transformé ainsi que de l'hydrogène moléculaire et/ou de la vapeur d'eau, et
- on prélève en continu de la chambre de réaction au moins un flux gazeux produit
**caractérisé**
**en ce que** la surface de la gaine est fabriquée, sur sa face en contact avec la chambre de réaction, au moins partiellement, en une épaisseur de couche d d'au moins 1 mm, en un acier S qui présente la composition élémentaire suivante : 18 à 30% en poids de Cr,
9 à 36% en poids de Ni,
1 à 3% en poids de Si,
0,1 à 0,3% en poids de N,
≥ 0 à 0,15% en poids de C,
≥ 0 à 4% en poids de Mn,
≥ 0 à 4% en poids d'Al,
≥ 0 à 0,05% en poids de P,
≥ 0 à 0,05% en poids de S et
≥ 0 à 0,1% en poids d'un ou de plusieurs métaux des terres rares et
pour le reste du Fe et des impuretés provoquées par la fabrication, les indications en pour cent se rapportant à chaque fois au poids total.

2. Procédé selon la revendication 1, **caractérisé en ce que** l'acier S présente la composition élémentaire suivants :
20 à 25% en poids de Cr
9 à 20% en poids de Ni,
1,4 à 2,5% en poids de Si,
0,1 à 0,3% en poids de N,
0,03 à 0,15% en poids de C,
≥ 0 à 3% en poids de Mn,
≥ 0 à 4% en poids d'Al,
≥ 0 à 0,05% en poids de P,
≥ 0 à 0,05% en poids de S et
≥ 0 à 0,1% en poids d'un ou de plusieurs métaux des terres rares et
pour le reste du Fe et des impuretés provoquées par la fabrication, les indications en pour cent se rapportant à chaque fois au poids total.

3. Procédé selon la revendication 1, **caractérisé en ce que** l'acier S présente la composition élémentaire suivants :
20 à 22% en poids de Cr,
10 à 12% en poids de Ni,
1,4 à 2,5% en poids de Si,
0,12 à 0,2% en poids de N,
0,05 à 0,12% en poids de C,
≥ 0 à 1% en poids de Mn,
≥ 0 à 2% en poids d'Al,
≥ 0 à 0,045% en poids de P,
≥ 0 à 0,015% en poids de S et
≥ 0,03 à 0,08% en poids de Ce ou de Ce et d'un ou de plusieurs métaux des terres rares et
pour le reste du Fe et des impuretés provoquées par la fabrication, les indications en pour cent se rapportant à chaque fois au poids total.

4. Procédé selon l'une quelconque des revendications 1 à 3, **caractérisé en ce que** l'hydrocarbure à déshydrogéner est un C₂-C₁₆-alcane.

5. Procédé selon l'une quelconque des revendications 1 à 3, **caractérisé en ce que** l'hydrocarbure à déshydrogéner est au moins un hydrocarbure du groupe comprenant l'éthane, le propane, le n-butane, l'isobutane, le n-pentane, l'isopentane, le n-hexane, le n-heptane, le n-octane, le n-nonane, le n-décane, le n-undécane, le n-dodécane, le n-tridécane, le n-tétradécane, le n-pentadécane et le n-hexadécane.

6. Procédé selon l'une quelconque des revendications 1 à 3, **caractérisé en ce que** l'hydrocarbure à déshydrogéner est l'éthane, le propane, le n-butane et/ou l'isobutane.

7. Procédé selon l'une quelconque des revendications 1 à 3, **caractérisé en ce que** l'hydrocarbure à déshydrogéner est le propane et l'hydrocarbure déshydrogéné est le propylène.

8. Procédé selon l'une quelconque des revendications 1 à 7, **caractérisé en ce que** le flux gazeux initial contient de la vapeur d'eau.

9. Procédé selon l'une quelconque des revendications 1 à 8, **caractérisé en ce que** le flux gazeux initial contient de l'oxygène moléculaire.

10. Procédé selon l'une quelconque des revendications 1 à 9, **caractérisé en ce que** le lit catalytique est un lit fixe catalytique.

11. Procédé selon l'une quelconque des revendications 1 à 10, **caractérisé en ce que** la surface de la gaine est fabriquée, sur sa face en contact avec la chambre de réaction, à raison d'au moins 10% de sa surface totale, en une épaisseur de couche d d'au moins 1 mm, en un acier S.

12. Procédé selon l'une quelconque des revendications 1 à 10, **caractérisé en ce que** la surface de la gaine est fabriquée, sur sa face en contact avec la chambre de réaction, à raison d'au moins 50% de sa surface totale, en une épaisseur de couche d d'au moins 1 mm, en un acier S.

13. Procédé selon l'une quelconque des revendications 1 à 12, **caractérisé en ce que** d vaut au moins 3 mm.

14. Procédé selon l'une quelconque des revendications 1 à 12, **caractérisé en ce que** d vaut au moins 5 mm.

15. Procédé selon l'une quelconque des revendications 1 à 14, **caractérisé en ce que** la gaine est fabriquée, à raison d'au moins 80% de son poids, en un acier S.

16. Procédé selon l'une quelconque des revendications 1 à 15, **caractérisé en ce que** la gaine est fabriquée dans sa totalité en un acier S.

17. Procédé selon l'une quelconque des revendications 1 à 16, **caractérisé en ce que** la déshydrogénation partielle catalysée par voie hétérogène est une déshydrogénation non oxydante.

18. Procédé selon l'une quelconque des revendications 1 à 16, **caractérisé en ce que** la déshydrogénation partielle catalysée par voie hétérogène est une déshydrogénation oxydante.

19. Procédé selon l'une quelconque des revendications 1 à 16, **caractérisé en ce que** la déshydrogénation partielle catalysée par voie hétérogène est une oxydéshydrogénation catalysée par voie hétérogène.

20. Procédé selon l'une quelconque des revendications 1 à 16, **caractérisé en ce que** la déshydrogénation partielle catalysée par voie hétérogène est une déshydrogénation catalysée par voie hétérogène, classique, réalisée de manière adiabatique.

21. Procédé selon l'une quelconque des revendications 1 à 16, **caractérisé en ce que** la déshydrogénation partielle catalysée par voie hétérogène est une déshydrogénation partielle catalysée par voie hétérogène, classique, et la chambre de réaction est une chambre de réaction à plateaux.

22. Procédé selon la revendication 21, **caractérisé en ce que** la déshydrogénation partielle catalysée par voie hétérogène, classique, est une déshydrogénation partielle, catalysée par voie hétérogène, classique, oxydante.

23. Procédé selon la revendication 22, **caractérisé en ce qu'**il est réalisé de manière adiabatique.

24. Procédé selon l'une quelconque des revendications 1 à 23, **caractérisé en ce que** le flux gazeux initial introduit dans la chambre de réaction contient :
du propylène à raison de ≥ 0 à 20% en volume,
de l'acroléine à raison de ≥ 0 à 1% en volume,
de l'acide acrylique à raison de ≥ 0 à 0,25% en volume,
du COₓ à raison de ≥ 0 à 20% en volume,
du propane à raison de 5 à 50% en volume,
de l'azote à raison de 20 à 80% en volume,
de l'oxygène à raison de ≥ 0 à 5% en volume,
H₂O à raison de ≥ 0 à 20% en volume et
H₂ à raison de ≥ 0 à 10% en volume.

25. Procédé selon l'une quelconque des revendications 1 à 24, **caractérisé en ce qu'**on utilise le flux gazeux produit prélevé de la chambre de réaction, tel quel ou après séparation d'au moins une quantité partielle de ses constituants différents de l'hydrocarbure déshydrogéné et de l'hydrocarbure à déshydrogéner, pour charger au moins un réacteur d'oxydation et on soumet dans celui-ci l'hydrocarbure déshydrogéné qui y est contenu à une oxydation en phase gazeuse partielle catalysée par voie hétérogène sélective avec de l'oxygène moléculaire en un mélange gazeux produit B contenant le produit d'oxydation partielle.

26. Procédé selon la revendication 25, **caractérisé en ce que** l'hydrocarbure à déshydrogéner est le propane, l'hydrocarbure déshydrogéné est le propylène et le produit d'oxydation partielle est l'acroléine, l'acide acrylique ou leur mélange.

27. Procédé selon la revendication 25, **caractérisé en ce que**, dans une zone de séparation B, consécutive à l'oxydation en phase gazeuse partielle catalysée par voie hétérogène, sélective, on sépare, du mélange gazeux produit B, un produit d'oxydation partielle et on recycle du gaz résiduel restant, contenant de l'hydrocarbure à déshydrogéner non transformé, de l'oxygène moléculaire ainsi que le cas échéant de l'hydrocarbure déshydrogéné non transformé, au moins une quantité partielle contenant de l'hydrocarbure à déshydrogéner non transformé, comme gaz en circulation d'oxydation partielle, dans le procédé de la déshydrogénation partielle catalysée par voie hétérogène de l'hydrocarbure à déshydrogéner.

28. Procédé selon la revendication 27, **caractérisé en ce qu'**on sépare le produit d'oxydation partielle, dans la zone de séparation B, du mélange gazeux produit B par transfert dans la phase condensée.

29. Procédé selon la revendication 28, **caractérisé en ce que** le produit d'oxydation partielle est l'acide acrylique et le transfert dans la phase condensée a lieu par des mesures d'absorption et/ou de condensation.

30. Procédé selon la revendication 29, **caractérisé en ce qu'**on réalise une séparation de l'acide acrylique de la phase condensée à l'aide d'au moins un procédé de séparation thermique.

31. Procédé selon la revendication 30, **caractérisé en ce que** ledit au moins un procédé de séparation thermique est une séparation par cristallisation de l'acide acrylique de la phase liquide.

32. Procédé selon la revendication 31, **caractérisé en ce que** la séparation par cristallisation est une cristallisation en suspension.

33. Procédé selon la revendication 30, **caractérisé en ce que** la séparation de l'acide acrylique est suivie d'un procédé de polymérisation par voie radicalaire, dans lequel l'acide acrylique séparé est polymérisé par voie radicalaire pour la préparation de polymères.

34. Procédé selon la revendication 30, **caractérisé en ce que** la séparation de l'acide acrylique est suivie d'un procédé de préparation d'esters de l'acide acrylique, dans lequel l'acide acrylique séparé est estérifié par un alcool.

35. Procédé selon la revendication 34, **caractérisé en ce que** le procédé de préparation d'un ester de l'acide acrylique est suivi d'un procédé de polymérisation par voie radicalaire dans lequel l'ester de l'acide acrylique ainsi préparé est polymérisé.

36. Gaine E entourant une chambre interne I, présentant au moins une première ouverture 01 pour l'introduction d'au moins un flux gazeux S dans la chambre interne I et au moins une deuxième ouverture 02 pour le prélèvement d'un flux gazeux S introduit au préalable dans la chambre interne I via ladite au moins une première ouverture 01 de la chambre interne I, la surface de la gaine E étant fabriquée, sur sa face en contact avec la chambre interne I, au moins partiellement, en une épaisseur de couche d d'au moins 1 mm, en un acier S, qui présente la composition élémentaire suivante :
18 à 30% en poids de Cr,
9 à 36% en poids de Ni,
1 à 3% en poids de Si,
0,1 à 0,3% en poids de N,
≥ 0 à 0,15% en poids de C,
≥ 0 à 4% en poids de Mn,
≥ 0 à 4% en poids d'Al,
≥ 0 à 0,05% en poids de P,
≥ 0 à 0,05% en poids de S et
≥ 0 à 0,1% en poids d'un ou de plusieurs métaux des terres rares et
pour le reste du Fe et des impuretés provoquées par la fabrication, les indications en pour cent se rapportant à chaque fois au poids total.

37. Gaine E selon la revendication 36, dont la chambre interne I contient au moins un catalyseur de déshydrogénation.

38. Gaine E selon la revendication 36 ou 37, dont la chambre interne I contient au moins une grille.

39. Gaine E selon l'une quelconque des revendications 36 à 38, qui présente un segment R annulaire.

40. Gaine E selon la revendication 39, le rapport V₁=D:A, formé à partir de la moitié D de la différence entre le diamètre externe A et le diamètre interne du segment annulaire R, valant de 1:10 à 1:1000.

41. Gaine E selon la revendication 40, V₁ valant de 1:40 à 1:500.

42. Gaine E selon l'une quelconque des revendications 39 à 41, le rapport V₂=H:A, formé à partir de la distance H des deux plans circulaires parallèles délimitant le segment annulaire R et du diamètre interne A du segment annulaire étant > 1.

43. Gaine E selon l'une quelconque des revendications 39 à 41, le rapport V₂=H:A, formé à partir de la distance H des deux plans circulaires parallèles délimitant le segment annulaire R et du diamètre interne du segment annulaire étant ≤ 1.

44. Gaine E selon l'une quelconque des revendications 36 à 38, qui présente un segment K en forme de zone sphérique creuse.

45. Gaine E selon l'une quelconque des revendications 36 à 44, qui présente un matériau isolant thermique appliqué sur la face opposée à la chambre interne I.

46. Procédé pour la déshydrogénation partielle catalysée par voie hétérogène d'un hydrocarbure, **caractérisé en ce qu'**on le réalise dans la chambre interne I d'une gaine E selon l'une quelconque des revendications 36 à 45.

47. Utilisation d'une gaine E selon l'une quelconque des revendications 36 à 45 pour réaliser une déshydrogénation partielle catalysée par voie hétérogène d'un hydrocarbure.

48. Procédé selon l'une quelconque des revendications 1 à 35, **caractérisé en ce que** l'acier S, sur sa face en contact avec la chambre de réaction, est alonisé, calorisé et/ou aluminisé.

49. Gaine E selon l'une quelconque des revendications 36 à 45, l'acier S étant alonisé, calorisé et/ou aluminisé sur sa face en contact avec la chambre interne I.
